(19) Europäisches Patentamt
European Patent Office
Office européen des brevets

(11) EP 1 031 629 A2

(12) # EUROPÄISCHE PATENTANMELDUNG

(43) Veröffentlichungstag:
**30.08.2000 Patentblatt 2000/35**

(51) Int. Cl.[7]: **C12P 41/00**, C12P 7/62
// C12N9/18

(21) Anmeldenummer: **00102505.5**

(22) Anmeldetag: **05.02.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **25.02.1999 DE 19908074**

(71) Anmelder:
**BASF AKTIENGESELLSCHAFT**
**67056 Ludwigshafen (DE)**

(72) Erfinder:
- **Bornscheuer, Uwe, Dr.**
  **17489 Greifswald (DE)**
- **Henke, Erik**
  **70180 Stuttgart (DE)**
- **Yang, Hong, Dr.**
  **O'Connor ACT 2602, Canberra (AU)**

## (54) Verfahren zur Herstellung von stereoisomeren Carbonsäureestern

(57) Die vorliegende Erfindung betrifft ein Verfahren zur Herstellung von stereoisomeren Carbonsäureestern bestehend aus einer Säure- und einer Alkohol-Komponente mit jeweils mindestens einem chiralen Zentrum, wobei mindestens ein Stereoisomer des Carbonsäureesters in einem Überschuß vorliegt, indem man einen racemischen Carbonsäureester mit einem racemischen Alkohol in Gegenwart einer Carboxylesterhydrolase (EC 3.1.1) umsetzt.



Printed by Xerox (UK) Business Services
2.16.7 (HRS)/3.6

## Beschreibung

Die Erfindung betrifft ein Verfahren zur Herstellung von stereoisomeren Carbonsäureestern bestehend aus einer Säure- und einer Alkohol-Komponente mit jeweils mindestens einem chiralen Zentrum, wobei mindestens ein Stereoisomer des Carbonsäureesters in einem Überschuß vorliegt.

Stereoisomerenreine Carbonsäureester mit mindestens zwei chiralen Zentren sind wichtige Vorstufen von biologisch aktiven Substanzen, wie Naturstoffen, Arzneimitteln und Pflanzenschutzmitteln. Ein wirtschaftliches Herstellungsverfahren ist deshalb von großer Bedeutung.

Es ist bekannt, optisch reine Alkohole durch Umesterung der racemischen Alkohole mit achiralen Carbonsäurealkylester in organischem Lösungsmittel in Gegenwart von Carboxylesterasen herzustellen (Klibanov et al., J. Am. Chem. Soc. 1984, 106, 2687-2692). Zur Erhöhung der Reaktionsrate und zur Verschiebung des Reaktionsgleichgewichtes hat es sich als sehr nützlich erwiesen, aktivierte achirale Carbonsäureester, insbesondere Enolester, Oximester oder Anhydride, einzusetzen. Häufig eingesetzte Acyldonoren sind Vinylester und hierunter vor allem Vinylacetat (siehe Degueil-Castaing et al., Tetrahedron Lett., 1987, 28, 953-954; Wang et al., J. Am. Chem. Soc., 1988, 110, 7200-7205; Laumen et al., J. Chem. Soc., Chem. Commun., 1988, 1459-1461), Oximester und hierunter vor allem 2-Propanonoximylacetat und Cyclohexanonoximylacetat (A. Ghogare, G.S. Kumar, J. Chem. Soc., Chem, Commun. 1989, 1533-1535; J. Chem. Soc., Chem. Commun. 1990, 134-135) oder Anhydride (D. Bianchi, P. Cesti, E. Battistel, J. Org. Chem. 1988, 53, 5531-5534; J.-H. Xu, T. Kawamoto, A. Tanaka, Appl. Microbiol. Biotechnol. 1995, 43, 639-643).

Weiterhin ist bekannt, optisch aktive Carbonsäuren durch Umsetzen ihrer racemischen Ester mit achiralen Alkoholen in organischen Lösungsmitteln in Gegenwart von Lipasen herzustellen (siehe Holmberg et al., Appl. Microbiol. Biotechnol., 1992, 35, 572-578; Persichetti et al., Tetrahedron Lett., 1996, 37, 6507-6510; Ozegowski et al., Liebigs Ann. 1994, 215-217).

Weiterhin ist bekannt, stereoisomerenangereicherte Amide durch Umsetzen von racemischen Ethyl-2-chlorpropionat mit racemischen Aminen in Gegenwart von Lipasen herzustellen (R. Brieva, J. Chem. Soc., Chem. Commun. 1990, 1386-1387).

Weiterhin ist bekannt, ein Gemisch von vier stereoisomeren Estern durch Umsetzung eines meso-Diols mit racemischem 2,2,2-Trifluoroethyl-2-chlorpropanoat in organischen Lösungsmitteln in Gegenwart einer Lipase herzustellen (F. Theil et al., Tetrahedron Lett. 1992, 33, 3457-3460). Das Verfahren hat den Nachteil, daß zwar kurze Reaktionszeiten, aber nur geringe Diastereomerenüberschüsse (3,6 %de bis 51,5 %de) erreicht werden.

Die Herstellung von stereoisomerenangereicherten Carbonsäureestern durch Umsatz von racemischen Carbonsäuren mit racemischen Alkoholen in organischen Lösungsmitteln in Gegenwart einer Lipase ist aus zwei Schriften bekannt: P.W. Fowler et al. beschreiben die Umsetzung von racemischer p-Chlorphenoxypropansäure mit einem racemischen Bicycloheptenol (J. Chem. Soc., Chem. Commun. 1991, 453-454). Das Verfahren hat den Nachteil, daß zwar höhere Diastereomerenüberschüsse (16 %de bis 72 %de), aber trotz hoher Reaktionszeiten nur ein geringer Umsatz und eine geringe Enantioselektivität (E(Alkohol) = 5,8 bis 26,7; E(Carbonsäure) = 1,3 bis 15,7) erreicht werden, wobei unter E die Enantioselektivität wie von Sih et al. definiert, verstanden wird (J. Am. Chem. Soc. 1982, 104, 7294-7299). Die Verwendung des acetylierten racemischen Bicycloheptenol führt zwar zu einer Selektivitätssteigerung, aber es werden trotz noch höherer Reaktionszeiten noch geringere Umsätze erreicht. Chen et al. beschreiben die Umsetzung von racemischen, chlorierten Phenoxypropionsäuren mit racemischen Phenylalkanolen. Das Verfahren hat den Nachteil, daß trotz hoher Reaktionszeiten nur geringe Umsätze und von einer Ausnahme (E(Säure) = 108) abgesehen nur geringe Enantioselektivitäten (E(Alkohol) = 1,0 bis 13,6; E(Carbonsäure) = 2,1 bis 35,6 erreicht werden. Daher werden auch nur geringe Diastereoselektivitäten erzielt.

Eine optimale und wirtschaftliche enzymkatalysierte Herstellung von stereoisomeren Carbonsäureestern bestehend aus einer Säure- und einer Alkoholkomponente mit jeweils mindestens einem chiralen Zentrum sollte vorteilhafterweise eine Reihe von Bedingungen erfüllen, wie beispielsweise

1. eine hohe Enantioselektivität, jeweils bezogen auf die Säure- und Alkoholkomponente,

2. eine hohe Diastereoselektivität,

3. gute Raum-Zeit-Ausbeute (kurze Reaktionszeiten, hohe Umsätze bezogen auf ein Enantiomer, hohe Edukt und Produktkonzentrationen),

4. hohe Substratbreite des Enzyms,

5. hohe chemische Ausbeute des gewünschten Produkts,

6. geringe Katalysatormengen (Enzymmengen),

7. leichte Reinigung der Syntheseprodukte,

8. gute Löslichkeit von Edukt und Produkt unter den Reaktionsbedingungen,

9. kostengünstige Synthese (leicht herstellbare Edukte, gute Handhabbarkeit der Edukte, Lösungsmittel, Reagenzien und Enzyme).

Der Erfindung lag daher die Aufgabe zugrunde, den geschilderten Mängeln des Standes der Technik abzuhelfen und ein verbessertes Verfahren bereitzustellen, das möglichst viele der vorstehend beschriebenen Bedingungen erfüllt.

Demgemäß wurde ein Verfahren zur Herstellung von stereoisomeren Carbonsäureestern, bestehend aus einer Carbonsäure- und einer Alkohol-Komponente mit jeweils mindestens einem chiralen Zentrum, wobei mindestens ein Stereoisomer des Carbonsäureesters in einem Überschuß vorliegt, gefunden in dem man einen racemischen Carbonsäureester mit einem racemischen Alkohol in Gegenwart einer Carboxylesterhydrolase (EC 3.1.1) (Enzyme Nomenclature 1992, NC-IUBMB, Academic Press, Inc., San Diago) umsetzt. Die Herstellung der stereoisomeren Carbonsäureester, wobei mindestens ein Stereoisomer des Carbonsäureesters in einem Überschuß vorliegt, erfolgt somit durch Umesterung eines racemischen Carbonsäureesters mit einem racemischen Alkohol in Gegenwart einer Carboxylesterhydrolase (EC 3.1.1).

Das erfindungsgemäße Verfahren führt zu einer vorteilhaften Steigerung der Reaktionsgeschwindigkeit und des Umsatzes bei gleichzeitiger Steigerung der Enantioselektivität und der Diastereoselektivität der verwendeten Enzyme (Carboxylesterhydrolasen). Es lassen sich so hohe Diastereomerenüberschüsse und Enantioselektivitäten bei hohen Reaktionsumsätzen und kurzen Reaktionszeiten erzielen.
Es werden bei ca. 50 % Umsatz Diastereomerenüberschüsse von mindestens 50 %de, bevorzugt mindestens 55 %de, erreicht. Die Enantioselektivität (E) für den Alkohol liegt vorteilhaft bei mindestens 30, bevorzugt bei 80, besonders bevorzugt bei größer 100.

Unter stereoisomeren Carbonsäureestern, bestehend aus einer Carbonsäure- und einer Alkohol-Komponente mit jeweils mindestens einem chiralen Zentrum, werden alle möglichen Stereoisomere eines Carbonsäureesters verstanden, wobei jeweils die Carbonsäure- und die Alkohol-Komponente mindestens ein chirales Zentrum enthalten.

Unter mindestens einem chiralen Zentrum werden 1 bis 3, bevorzugt 1 oder 2, chirale Zentren, besonders bevorzugt ein chirales Zentrum jeweils für die Carbonsäure- und die Alkohol-Komponente verstanden. Für i chirale Zentren im Gesamtmolekül des Carbonsäureesters ergeben sich $2^i$ mögliche Stereoisomere des Carbonsäureesters. Dementsprechend weisen der racemische Carbonsäureester wie auch der racemische Alkohol jeweils mindestens ein chirales Zentrum auf. Prinzipiell sind racemische Alkohole und racemische Carbonsäureester, die jeweils mindestens ein chirales Zentrum aufweisen, mit einer großen strukturellen Breite dem erfindungsgemäßen Verfahren zugänglich.
In einer bevorzugten Ausführungsform des Verfahrens verwendet man als racemischen Carbonsäureester einen aktivierten racemischen Carbonsäureester. Unter einem aktivierten racemischen Carbonsäureester wird ein racemischer Carbonsäureester verstanden, der eine Komponente mit Hydroxylgruppe als Abgangsgruppe enthält, die bei der Übertragung der racemischen Acylgruppe (Säure-Komponente) des racemischen Carbonsäureesters auf einen Alkohol bessere Abgangsgruppeneigenschaften als Ethanol aufweist. Die Abgangsgruppe der aktivierten racemischen Carbonsäureester weist demnach eine geringere Nukleophilie als Ethanol auf. Beispielhaft seien als aktivierte racemische Carbonsäureester racemische Carbonsäurehalogenalkylester, wie Carbonsäuretrichlorethylester oder Carbonsäuretrifluorethylester oder racemische Carbonsäurehalogenarylester wie Carbonsäurepentachlorphenylester oder Anhydride erwähnt, wobei bei Anhydriden die entsprechende racemische Carbonsäure die Abgangsgruppe darstellt.
Unter aktivierten racemischen Carbonsäureestern werden auch racemische Carbonsäureester verstanden, bei denen sich die Abgangsgruppe nach der Freisetzung umlagert oder auf eine andere Weise nicht mehr als Reaktionspartner der Gleichgewichtsreaktion zur Verfügung steht, so daß die Rückreaktion unterdrückt wird und das Gleichgewicht in die gewünschte Richtung gelenkt wird. Beispielhaft seien als aktivierte racemische Carbonsäureester racemische Carbonsäureenolester, wie Carbonsäurevinylester, Carbonsäure(1-methyl)-vinylester, Carbonsäure-(1-ethyl)-vinylester, Carbonsäure (1-propyl)-vinylester oder Carbonsäure (1-butyl)-vinylester oder racemische Oximester, wie Carbonsäure-2-Propanoximylester oder Carbonsäure-cyclohexanonoximylester erwähnt. Vorzugsweise verwendet man als aktivierte racemische Carbonsäureester racemische Carbonsäureenolester, insbesondere Carbonsäurevinylester oder racemische Carbonsäure-(1-methyl)-vinylester.

In einer bevorzugten Ausführungsform des erfindungsgemäßen Verfahrens weisen die Säure- und die Alkoholkomponente der stereoisomeren Carbonsäureester und dementsprechend der racemische Carbonsäureester und der racemische Alkohol jeweils ein chirales Zentrum auf. Dementsprechend entstehen aufgrund der zwei chiralen Zentren im Gesamtmolekül der stereoisomeren Carbonsäureester vier mögliche Stereoisomere des Carbonsäureesters (R,R), (S,S), (R,S) und (S,R) (In jeder Klammer gibt der erste Buchstabe die absolute Konfiguration (nach Cahn-Ingold-Prelog) des chiralen Zentrums der Carbonsäurekomponente und der zweite Buchstabe die absolute Konfiguration des

chiralen Zentrums der Alkoholkomponente an), wobei mindestens ein, in diesem Fall vorzugsweise ein Stereoisomer, im Überschuß entsteht (doppelte Enantioselektivität). Unter Überschuß eines Stereoisomer wird in diesem Fall verstanden, wenn der Anteil des Stereoisomer im Stereoisomerengemisch größer als 25 % ist (Molverhältnis). Unter Diastereomerenüberschuß wird in diesem Fall der Überschuß der Summe der Anteile des (R,R)- und des (S,S)-Stereoisomer gegenüber der Summe der Anteile des (S,R)- und des (R,S)-Stereoisomer bzw. der Überschuß der Summe der Anteile des (S,R)- und des (R,S)-Stereoisomer gegenüber der Summe der Anteile des (R,R)- und des (S,S)-Stereoisomer verstanden, je nachdem welches Stereoisomer im Überschuß vorliegt.

In einer besonders bevorzugten Ausführungsform des erfindungsgemäßen Verfahrens werden stereoisomere Carbonsäureester der allgemeinen Formel I

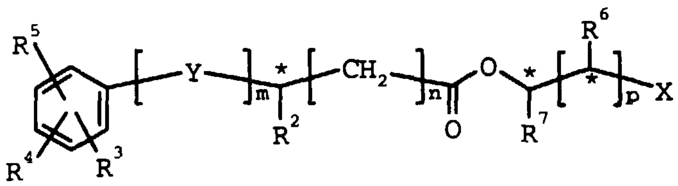 (I),

wobei die Substituenten und Variablen

| | |
|---|---|
| * | ein mögliches chirales Zentrum, |
| n, m, p | unabhängig voneinander 0 oder 1, |
| X | einen unsubstituierten oder mit F, Cl, Br, I, $NO_2$ oder $C_1$-$C_4$-Alkoxy substituierten $C_1$-$C_{10}$-Alkyl-, $C_2$-$C_{10}$-Alkenyl-, $C_2$-$C_{10}$-Alkinyl- oder $C_3$-$C_8$-Cycloalkenyl-, $C_6$-$C_{14}$-Aryl-, $C_7$-$C_{16}$-Alkylaryl-, $C_8$-$C_{16}$-Alkenylaryl-, $C_8$-$C_{16}$-Alkinylaryl-, $C_7$-$C_{18}$-Arylalkyl-, $C_8$-$C_{18}$-Arylalkenyl-, $C_8$-$C_{18}$-Arylalkinyl- oder $C_4$-$C_{12}$-Heteroarylrest oder einen substituierten $C_6$-$C_{14}$-Aryl-, $C_7$-$C_{16}$-Alkylaryl-, $C_8$-$C_{16}$-Alkenylaryl-, $C_8$-$C_{16}$-Alkinylaryl-, $C_7$-$C_{18}$-Arylalkyl-, $C_8$-$C_{18}$-Arylalkenyl-, $C_8$-$C_{18}$-Arylalkinyl oder $C_4$-$C_{12}$-Heteroarylrest, wobei jeweils zwei benachbarte Arylsubstituenten zusammen einen weiteren substituierten oder unsubstituierten aromatischen, gesättigten oder teilweise gesättigten Ring mit 5 bis 6 Atomen im Ring bilden können, der ein oder mehrere Heteroatome wie O, N, oder S enthalten kann, |
| Y | -$CH_2$-, -CO-, Sauerstoff, oder Schwefel, |
| $R^2$ | einen substituierten oder unsubstituierten, verzweigten oder unverzweigten $C_1$-$C_6$-Alkyl-, $C_1$-$C_4$-Alkoxy-, $C_3$-$C_6$-Cycloalkyl-, $C_6$-$C_{14}$-Aryl- oder $C_4$-$C_{12}$-Heteroarylrest, |
| $R^3$, $R^4$, $R^5$ | unabhängig voneinander Wasserstoff, einen Hydroxy-, Halogen-, Cyano-, Nitro- oder Aminorest oder einen substituierten oder unsubstituierten, verzweigten oder unverzweigten $C_1$-$C_{10}$-Alkyl-, $C_2$-$C_{10}$-Alkenyl-, $C_2$-$C_{10}$-Alkinyl-, $C_1$-$C_{10}$-Alkoxy-, $C_2$-$C_{10}$-Alkenyloxy-, $C_2$-$C_{10}$-Alkinyloxy-, $C_3$-$C_{10}$-Cycloalkyl-, $C_3$-$C_{10}$-Cycloalkyloxy-, $C_6$-$C_{14}$-Aryl-, $C_7$-$C_{18}$-Alkylaryl-, $C_5$-$C_{16}$-Alkylheteroaryl- oder $C_4$-$C_{12}$-Heteroarylrest oder einen substituierten oder unsubstituierten olefinischen oder aromatischen Acylrest oder zwei benachbarte Substituenten $R^3$, $R^4$ oder $R^5$ zusammen einen weiteren substituierten oder unsubstituierten aromatischen, gesättigten oder teilweise gesättigten Ring mit 5 bis 6 Atomen, der ein oder mehrere Heteroatome wie O, N oder S enthalten kann, |
| $R^6$ | Wasserstoff oder einen substituierten oder unsubstituierten, verzweigten oder unverzweigten $C_1$-$C_6$-Alkyl-, $C_1$-$C_4$-Alkoxy-, $C_3$-$C_6$-Cycloalkyl-, $C_6$-$C_{14}$-Aryl- oder $C_4$-$C_{12}$-Heteroarylrest und |
| $R^7$ | Wasserstoff oder einen substituierten oder unsubstituierten, verzweigten oder unverzweigten $C_1$-$C_6$-Alkyl-, $C_1$-$C_4$-Alkoxy- oder $C_3$-$C_6$-Cycloalkylrest bedeuten, |

wobei für den Fall, daß p = 0, $R^7$ nicht Wasserstoff oder X und für den Fall, daß p = 1, entweder $R^6$ oder $R^7$ Wasserstoff, aber nicht beide gleichzeitig Wasserstoff und für den Fall, daß $R^7$ = Wasserstoff, $R^6$ nicht X und für den Fall, daß $R^7$ nicht Wasserstoff, $R^6$ Wasserstoff oder X bedeuten,
herstellt, indem man einen racemischen Carbonsäureester der allgemeinen Formel II

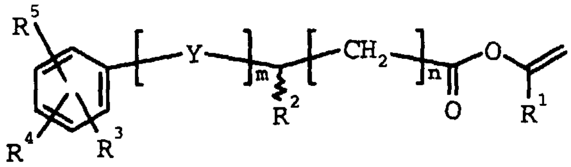

(II),

wobei

$R^1$ Wasserstoff oder $C_1$-$C_4$-Alkyl bedeutet,

mit einem racemischen Alkohol der allgemeinen Formel III

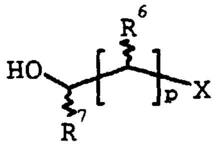

(III)

in Gegenwart einer Carboxylesterhydrolase (EC 3.1.1) umsetzt.

Die mit * gekennzeichneten möglichen chiralen Zentren können unabhängig voneinander entweder in der R- oder S-Konfiguration (nach Cahn-Ingold-Prelog) vorliegen. Bei dieser besonders bevorzugten Ausführungsform des erfindungsgemäßen Verfahrens, weisen die racemischen Carbonsäureester der Formel II und die racemischen Alkohole der Formel III und damit auch jeweils die Säure- und Alkoholkomponente der stereoisomeren Carbonsäureester der Formel I, wie vorstehend erwähnt, jeweils nur ein chirales Zentrum auf.

Die Variablen n, m und p bedeuten unabhängig voneinander 0 oder 1. Um zu Erreichen, daß in der Alkoholkomponente des stereoisomeren Carbonsäureesters der Formel I nur ein chirales Zentrum vorliegt, gilt für den Fall p = O, daß $R^7$ nicht Wasserstoff oder X bedeutet und für den Fall p = 1, daß entweder $R^6$ oder $R^7$ Wasserstoff, aber nicht beide gleichzeitig Wasserstoff bedeuten und für den Fall, daß $R^7$ = Wasserstoff ist, $R^6$ nicht X und für den Fall, daß $R^7$ nicht Wasserstoff ist, $R^6$ Wasserstoff oder X bedeuten.

Unter X werden unsubstituierte oder mit F, Cl, Br, I, $NO_2$ oder $C_1$-$C_4$-Alkoxy, wie Methoxy, Ethoxy, Propoxy, Isopropoxy, n-Butoxy, sec.-Butoxy oder t.-Butoxy, vorzugsweise Methoxy substituierte

$C_1$-$C_{10}$-Alkylreste, wie beispielsweise
Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl, n-Hexyl, n-Heptyl, n-Octyl, n-Nonyl, n-Dekayl, vorzugsweise Ethyl, Propyl oder Butyl oder $C_2$-$C_{10}$-Alkenylreste, wie beispielsweise Vinyl, 1-Propenyl, 1-Butenyl, 1-Pentenyl, 1-Hexenyl, 1-Heptenyl, 1-Octenyl, 1-Nonenyl, 1-Decenyl, 2-Chlorvinyl, 3-Chlor-1-propenyl, 4-Chlor-1-Butenyl, 5-Chlor-1-Pentenyl, 6-Chlor-1-Hexenyl, 7-Chlor-1-Heptenyl, 8-Chlor-1-Octenyl, 9-Chlor-1-Nonenyl, 10-Chlor-1-Decenyl, 2-Nitrovinyl, 3-Nitro-1-propenyl, 4-Nitro-1-Butenyl, 5-Nitro-1-Pentenyl, 6-Nitro-1-Hexenyl, 7-Nitro-1-Heptenyl, 8-Nitro-1-Octenyl, 9-Nitro-1-Nonenyl oder 10-Nitro-1-Decenyl, vorzugsweise Vinyl, 1-Propenyl, 1-Butenyl, 1-Pentenyl oder 1-Hexenyl oder

$C_2$-$C_{10}$-Alkinylreste, wie beispielsweise
Ethinyl, 1-Propinyl, 1-Butinyl, 1-Pentinyl, 1-Hexinyl, 1-Heptinyl, 1-Octinyl, 1-Noninyl, 1-Decinyl, 2-Chlorvinyl, 3-Chlor-1-propinyl, 4-Chlor-1-butinyl, 5-Chlor-1-pentinyl, 6-Chlor-1-hexinyl, 7-Chlor-1-heptinyl, 8-Chlor-1-octinyl, 9-Chlor-1-noninyl, 10-Chlor-1-decinyl, 2-nitrovinyl, 3-Nitro-1-propinyl, 4-Nitro-1-butinyl, 5-Nitro-1-pentinyl, 6-Nitro-1-hexinyl, 7-Nitro-1-heptinyl, 8-Nitro-1-octinyl, 9-Nitro-1-noninyl oder 10-Nitro-1-decinyl, vorzugsweise Ethinyl, 1-Propinyl, 1-Butinyl, 1-Pentinyl oder 1-Hexinyl oder

$C_3$-$C_8$-Cycloalkenylreste, wie beispielsweise
Cyclopropenyl, Cyclobutenyl, Cyclopentenyl, Cyclohexenyl, Cycloheptenyl, Cyclooctenyl, vorzugsweise Cyclopen-

tenyl oder Cyclohexenyl oder

$C_6$-$C_{14}$-Arylreste, wie beispielsweise
Phenyl, Naphthyl, Anthracyl, Phenanthryl, p-Nitrophenyl, o-Nitrophenyl, m-Nitrophenyl, p-Chlorphenyl, o-Chlorphenyl, m-Chlorphenyl, 2,4-Dichlorphenyl, 2,3-Dichlorphenyl, 3,5-Dichlorphenyl, 3,4-Dichlorphenyl, 2,4-Dimethoxyphenyl, 2,3-Dimethoxyphenyl, 3,5-Dimethoxyphenyl, 3,4-Dimethoxyphenyl, 2-Chlor-4-Nitrophenyl, p-Methoxyphenyl, o-Methoxyphenyl, m-Methoxyphenyl oder 6-Methoxy-2-naphthyl, vorzugsweise Phenyl, p-Nitrophenyl, p-Chlorphenyl oder 6-Methoxy-2-naphthyl oder

$C_7$-$C_{16}$-Alkylarylreste, wie beispielsweise
Benzyl, p-Chlorbenzyl, p-Nitrobenzyl, p-Methoxybenzyl, 2-Phenylethyl, 3-Phenylpropyl, 2-(p-Nitrophenyl)ethyl, 2-(p-Chlorphenyl)ethyl oder 2-(p-Methoxyphenyl)ethyl, vorzugsweise Benzyl, p-Chlorbenzyl oder p-Methoxybenzyl oder

$C_8$-$C_{16}$-Alkenylarylreste, wie beispielsweise
2-Phenylvinyl, 2-(p-Nitrophenyl)vinyl, 2-(p-Chlorphenyl)vinyl oder 2-(p-Methoxyphenyl)vinyl oder 2-Benzylvinyl, vorzugsweise 2-Phenylvinyl oder

$C_8$-$C_{16}$-Alkinylarylreste, wie beispielsweise
2-Phenylethinyl, 2-(p-Nitrophenyl)ethinyl, 2-(p-Chlorphenyl)ethinyl oder 2-(p-Methoxyphenyl)ethinyl, vorzugsweise 2-Phenylethinyl oder

$C_7$-$C_{18}$-Arylalkylreste, wie beispielsweise
p-Methylphenyl, p-Ethylphenyl, p-Propylphenyl, p-Isopropylphenyl, p-t-Butylphenyl, o-Methylphenyl, o-Ethylphenyl, o-Propylphenyl, o-Isopropylphenyl, o-t.-Butylphenyl, 4-Chlor-2-Methylphenyl oder 4-Methoxy-2-Methylphenyl, vorzugsweise p-Methylphenyl oder

$C_8$-$C_{18}$-Arylalkenylreste, wie beispielsweise
p-Vinylphenyl, oder

$C_8$-$C_{18}$-Arylalkinylreste, wie beispielsweise
p-Ethinylphenyl, oder

$C_4$-$C_{12}$-Heteroarylreste, wie beispielsweise
Furyl, Thienyl, Pyrrolyl, Pyridyl, Pyrimidyl, Pyrazinyl, Imidazolyl, Oxazolyl, Thiazolyl, Indolyl, 2-(4-Chlor)thienyl, vorzugsweise Furyl, Thienyl, Pyrrolyl, Pyridyl verstanden.

Ferner werden unter X substituierte $C_6$-$C_{14}$-Aryl-, $C_7$-$C_{16}$-Alkylaryl-, $C_8$-$C_{16}$-Alkenylaryl-, $C_8$-$C_{16}$-Alkinylaryl-, $C_7$-$C_{18}$-Arylalkyl-, $C_8$-$C_{18}$-Arylalkenyl-, $C_8$-$C_{18}$-Arylalkinyl oder $C_4$-$C_{12}$-Heteroarylrest verstanden, wobei jeweils zwei benachbarte Arylsubstituenten zusammen einen weiteren substituierten oder unsubstituierten aromatischen, gesättigten oder teilweise gesättigten Ring mit 5 bis 6 Atomen im Ring bilden können, der ein oder mehrere Heteroatome wie O, N, oder S enthalten kann. Beispielhaft seien folgende Strukturen aufgezählt, wobei die Verknüfungsstellen durch einen Bindungsstrich gekennzeichnet sind:

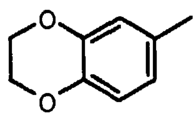
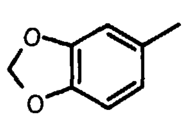
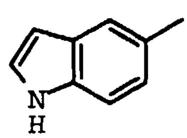

$R^2$ bedeutet einen substituierten oder unsubstituierten, verzweigten oder unverzweigten $C_1$-$C_6$-Alkyl-, $C_3$-$C_6$-Cycloalkyl-, $C_1$-$C_4$-Alkoxy-, $C_6$-$C_{14}$-Aryl- oder $C_4$-$C_{12}$-Heteroarylrest.

Als unsubstituierte, verzweigte oder unverzweigte $C_1$-$C_6$-Alkylreste für $R^2$ seien beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl genannt. Bevorzugt sind Methyl, Ethyl, n-Propyl, n-Butyl, i-Propyl oder i-Butyl. Als substituierte $C_1$-$C_6$-Alkylreste seien beispielhaft die entsprechenden, mit den nachstehend beschriebenen Substituenten, substituierten $C_1$-$C_6$-Alkylreste genannt.

Als unsubstituierte, verzweigte oder unverzweigte $C_3$-$C_6$-Cycloalkylreste für $R^2$ seien beispielsweise Cyclopropyl, Cyclobutyl, Cyclopentyl, Cyclohexyl, 1-Methylcyclopropyl, 1-Ethylcyclopropyl oder 1-Propylcyclopropyl genannt. Die Cycloalkylreste können auch Heteroatome wie S, N und O im Ring enthalten. Als substituierte $C_3$-$C_6$-Cycloalkylreste seien beispielhaft die entsprechenden, mit den nachstehend beschriebenen Substituenten, substituierten $C_3$-$C_6$-Cycloalkylreste genannt.

Als unsubstituierte, verzweigte oder unverzweigte $C_1$-$C_4$-Alkoxyreste für $R^2$ seien beispielsweise Methoxy, Ethoxy, Propoxy, Isopropoxy, n-Butoxy, sec.-Butoxy oder t-Butoxy, vorzugsweise Methoxy, genannt. Als substituierte $C_1$-$C_4$-Alkoxyreste seien beispielhaft die entsprechenden, mit den nachstehend beschriebenen Substituenten, substituierten $C_1$-$C_4$-Alkoxyreste genannt.

Als unsubstituierte oder substituierte $C_6$-$C_{14}$-Arylreste oder $C_4$-$C_{12}$-Heteroarylreste für $R^2$ seien die für X beispielhaft beschriebenen $C_6$-$C_{14}$-Arylreste bzw. $C_4$-$C_{12}$-Heteroarylreste, vorzugsweise Phenyl, Naphthyl, p-Chlorphenyl, p-Methoxyphenyl oder Furyl, genannt.

Als Substituenten der genannten Reste von $R^2$ kommen beispielsweise ein oder mehrere Substituenten wie Halogen, wie Fluor, Chlor oder Brom oder Cyano, Nitro, Amino oder Hydroxy in Frage. Bevorzugt sind Methyl, Chlor oder Hydroxy.

$R^3$, $R^4$, $R^5$ bedeuten unabhängig voneinander Wasserstoff oder einen Hydroxyl-, Cyano-, Nitro-, Amino- oder Halogenrest, wie Fluor, Chlor, Brom oder Iod, oder
einen substituierten oder unsubstituierten, verzweigten oder unverzweigten $C_1$-$C_{10}$-Alkyl-, $C_2$-$C_{10}$-Alkenyl-, $C_2$-$C_{10}$-Alkinyl-, $C_1$-$C_{10}$-Alkoxy-, $C_2$-$C_{10}$-Alkenyloxy-, $C_2$-$C_{10}$-Alkinyloxy-, $C_3$-$C_{10}$-Cycloalkyl-, $C_3$-$C_{10}$-Cycloalkyloxy-, $C_4$-$C_{14}$-Aryl-, $C_7$-$C_{18}$-Alkylaryl-, $C_4$-$C_{12}$-Hetaryl- oder $C_5$-$C_{16}$-Alkylheteroarylrest oder einen substituierten oder unsubstituierten olefinischen oder aromatischen Acylrest.
Ferner können zwei benachbarte Substituenten $R^3$, $R^4$ oder $R^5$ zusammen einen weiteren substituierten oder unsubstituierten aromatischen, gesättigten oder teilweise gesättigten Ring mit 5 bis 6 Atomen im Ring bilden, der ein oder mehrere Heteroatome wie O, N oder S enthalten kann. Dabei können kondensierte Systeme entstehen, wobei nicht mehr als ein Ring an den zentralen Ring ankondensiert sein kann, wie substituiertes oder unsubstituiertes Naphthyl. Als Substituenten kommen in diesem Fall vorzugsweise Halogenreste, wie Fluor, Brom oder Iod oder $C_1$-$C_4$-Alkoxyreste, wie Methoxy, Ethoxy, n-Propoxy, Isopropoxy, n-Butoxy, iso-Butoxy oder tert.-Butoxy, in Frage.

Als unsubstituierte, verzweigte oder unverzweigte $C_1$-$C_{10}$-Alkylreste seien für $R^3$, $R^4$ oder $R^5$ beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl oder n-Decyl genannt. Bevorzugt sind Methyl, Ethyl, n-Propyl, n-Butyl, i-Propyl oder i-Butyl. Als substituierte $C_1$-$C_{10}$-Alkylreste seien beispielhaft die entsprechenden, mit den nachstehend beschriebenen Substituenten, substituierten $C_1$-$C_{10}$-Alkylreste genannt.

Als unsubstituierte, verzweigte oder unverzweigte $C_2$-$C_{10}$-Alkenylreste seien für $R^3$, $R^4$ oder $R^5$ beispielsweise Ethenyl (Vinyl), Propenyl, 1-Butenyl, 2-Butenyl, 3-Butenyl, 2-Methylpropenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl, 1-Heptenyl, 2-Heptenyl, 3-Heptenyl, 4-Heptenyl, 5-Heptenyl, 6-Heptenyl, 1-Octenyl, 2-Octenyl, 3-Octenyl, 4-Octenyl, 5-Octenyl, 6-Octenyl, 7-Octenyl, 1-Nonenyl, 2-Nonenyl, 3-Nonenyl, 4-Nonenyl, 5-Nonenyl, 6-Nonenyl, 7-Nonenyl, 8-Nonenyl, 1-Decenyl, 2-Decenyl, 3-Decenyl, 4-Decenyl, 5-Decenyl, 6-Decenyl, 7-Decenyl, 8-Decenyl oder 9-Decenyl genannt. Als substituierte $C_2$-$C_{10}$-Alkenylreste seien beispielhaft die entsprechenden, mit den nachstehend beschriebenen Substituenten, substituierten $C_2$-$C_{10}$-Alkenylreste genannt.

Als unsubstituierte, verzweigte oder unverzweigte $C_2$-$C_{10}$-Alkinylreste seien für $R^3$, $R^4$ oder $R^5$ beispiels-

weise Ethinyl-, Prop-1-in-1-yl, Prop-2-in-1-yl, n-But-1-in-1-yl, n-But-1-in-3-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl oder 4-Methyl-pent-2-in-5-yl und die höheren Homologen dieser Reihe genannt. Als substituierte $C_2$-$C_{10}$-Alkinylreste seien beispielhaft die entsprechenden, mit den nachstehend beschriebenen Substituenten, substituierten $C_2$-$C_{10}$-Alkinylreste genannt.

Als unsubstituierte, verzweigte oder unverzweigte $C_1$-$C_{10}$-Alkoxyreste seien für $R^3$, $R^4$ oder $R^5$ beispielsweise Methoxy, Ethoxy, Propoxy, 1-Methylethoxy, Butoxy, 1-Methylpropoxy, 2-Methylpropoxy, 1,1-Dimethylethoxy, Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy, 1-Ethyl-2-methylpropoxy, Hexyloxy, Heptyloxy, Octyloxy, Nonyloxy oder Decyloxy und deren verzweigtkettige Homologen genannt. Als substituierte $C_1$-$C_{10}$-Alkoxyreste seien beispielhaft die entsprechenden, mit den nachstehend beschriebenen Substituenten, substituierten $C_1$-$C_{10}$-Alkoxyreste genannt.

Als unsubstituierte, verzweigte oder unverzweigte $C_2$-$C_{10}$-Alkenyloxyreste seien für $R^3$, $R^4$ oder $R^5$ beispielsweise Ethenyloxy, Propenyloxy, 1-Butenyloxy, 2-Butenyloxy, 3-Butenyloxy, 2-Methylpropenyloxy, 1-Pentenyloxy, 2-Pentenyloxy, 3-Pentenyloxy, 4-Pentenyloxy, 1-Methyl-1-butenyloxy, 2-Methyl-1-butenyloxy, 3-Methyl-1-butenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-1-propenyloxy, 1,2-Dimethyl-2-propenyloxy, 1-Ethyl-1-propenyloxy, 1-Ethyl-2-propenyloxy, 1-Hexenyloxy, 2-Hexenyloxy, 3-Hexenyloxy, 4-Hexenyloxy, 5-Hexenyloxy, 1-Methyl-1-pentenyloxy, 2-Methyl-1-pentenyloxy, 3-Methyl-1-pentenyloxy, 4-Methyl-1-pentenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy, 1,2-Dimethyl-1-butenyloxy, 1,2-Dimethyl-2-butenyloxy, 1,2-Dimethyl-3-butenyloxy, 1,3-Di-methyl-1-butenyloxy, 1,3-Dimethyl-2-butenyloxy, 1,3-Dimethyl-3-butenyloxy, 2,2-Dimethyl-3-butenyloxy, 2,3-Dimethyl-1-butenyloxy, 2,3-Dimethyl-2-butenyloxy, 2,3-Dimethyl-3-butenyloxy, 3,3-Dimethyl-1-butenyloxy, 3,3-Dimethyl-2-butenyloxy, 1-Ethyl-1-butenyloxy, 1-Ethyl-2-butenyloxy, 1-Ethyl-3-butenyloxy, 2-Ethyl-1-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy, 1-Ethyl-2-methyl-1-propenyloxy, 1-Ethyl-2-methyl-2-propenyloxy, 1-Heptenyloxy, 2-Heptenyloxy, 3-Heptenyloxy, 4-Heptenyloxy, 5-Heptenyloxy, 6-Heptenyloxy, 1-Octenyloxy, 2-Octenyloxy, 3-Octenyloxy, 4-Octenyloxy, 5-Octenyloxy, 6-Octenyloxy, 7-Octenyloxy, 1-Nonenyloxy, 2-Nonenyloxy, 3-Nonenyloxy, 4-Nonenyloxy, 5-Nonenyloxy, 6-Nonenyloxy, 7-Nonenyloxy, 8-Nonenyloxy, 1-Decenyloxy, 2-Decenyloxy, 3-Decenyloxy, 4-Decenyloxy, 5-Decenyloxy, 6-Decenyloxy, 7-Decenyloxy, 8-Decenyloxy oder 9-Decenyloxy genannt. Als substituierte $C_2$-$C_{10}$-Alkenyloxyreste seien beispielhaft die entsprechenden, mit den nachstehend beschriebenen Substituenten, substituierten $C_2$-$C_{10}$-Alkenyloxyreste genannt.

Als unsubstituierte, verzweigte oder unverzweigte $C_2$-$C_{10}$-Alkinyloxyreste seien für $R^3$, $R^4$ oder $R^5$ beispielsweise wie beispielsweise Ethinyloxy-, Prop-1-in-1-yloxy, Prop-2-in-1-yloxy, n-But-1-in-1-yloxy, n-But-1-in-3-yloxy, n-But-1-in-4-yloxy, n-But-2-in-1-yloxy, n-Pent-1-in-1-yloxy, n-Pent-1-in-3-yloxy, n-Pent-1-in-4-yloxy, n-Pent-1-in-5-yloxy, n-Pent-2-in-1-yloxy, n-Pent-2-in-4-yloxy, n-Pent-2-in-5-yloxy, 3-Methyl-but-1-in-3-yloxy, 3-Methyl-but-1-in-4-yloxy, n-Hex-1-in-1-yloxy, n-Hex-1-in-3-yloxy, n-Hex-1-in-4-yloxy, n-Hex-1-in-5-yloxy, n-Hex-1-in-6-yloxy, n-Hex-2-in-1-yloxy, n-Hex-2-in-4-yloxy, n-Hex-2-in-5-yl, n-Hex-2-in-6-yloxy, n-Hex-3-in-1-yloxy, n-Hex-3-in-2-yloxy, 3-Methyl-pent-1-in-1-yloxy, 3-Methylpent-1-in-3-yloxy, 3-Methyl-pent-1-in-4-yloxy, 3-Methylpent-1-in-5-yloxy, 4-Methyl-pent-1-in-1-yloxy, 4-Methyl-pent-2-in-4-yloxy oder 4-Methyl-pent-2-in-5-yloxy und die höheren Homologen dieser Reihe genannt. Als substituierte $C_2$-$C_{10}$-Alkinyloxyreste seien beispielhaft die entsprechenden, mit den nachstehend beschriebenen Substituenten, substituierten $C_2$-$C_{10}$-Alkinyloxyreste genannt.

Als unsubstituierte, verzweigte oder unverzweigte $C_3$-$C_{10}$-Cycloalkylreste seien für $R^3$, $R^4$ oder $R^5$ beispielsweise Cyclopropyl, Cyclobutyl, Cyclopentyl, Cyclohexyl, Cycloheptyl, 1-Methylcyclopropyl, 1-Ethylcyclopropyl, 1-Propylcyclopropyl, 1-Butylcyclopropyl, 1-Pentylcyclopropyl, 1-Methyl-1-Butylcyclopropyl, 1,2-Dimethylcyclypropyl, 1-Methyl-2-Ethylcyclopropyl, Cyclooctyl, Cyclononyl oder Cyclodecyl genannt. Die Cycloalkylreste können auch Heteroatome wie S, N und O im Ring enthalten. Als substituierte $C_3$-$C_{10}$-Cycloalkylreste seien beispielhaft die entsprechenden, mit den nachstehend beschriebenen Substituenten, substituierten $C_3$-$C_{10}$-Cycloalkylreste genannt.

Als unsubstituierte, verzweigte oder unverzweigte $C_3$-$C_{10}$-Cycloalkyloxyreste seien für $R^3$, $R^4$ oder $R^5$ beispielsweise Cyclopropyloxy, Cyclobutyloxy, Cyclopentyloxy, Cyclohexyloxy, Cycloheptyloxy, 1-Methylcyclopropyloxy, 1-Ethylcyclopropyloxy, 1-Propylcyclopropyloxy, 1-Butylcyclopropyloxy, 1-Pentylcyclopropyloxy, 1-Methyl-1-Butylcyclopropyloxy, 1,2-Dimethylcyclypropyloxy, 1-Methyl-2-Ethylcyclopropyloxy, Cyclooctyloxy, Cyclononyloxy oder Cyclodecyloxy

genannt. Die Cycloalkyloxyreste können auch weitere Heteroatome wie S, N und O im Ring enthalten. Als substituierte $C_3$-$C_{10}$-Cycloalkyloxyreste seien beispielhaft die entsprechenden, mit den nachstehend beschriebenen Substituenten, substituierten $C_3$-$C_{10}$-Cycloalkyloxyreste genannt.

Als unsubstituierte, verzweigtkettige oder unverzweigtkettige $C_7$-$C_{16}$-Alkylarylreste seien für $R^3$, $R^4$ oder $R^5$ beispielsweise Methylphenyl, Ethylphenyl, Propylphenyl, 1-Methylethylphenyl, Butylphenyl, 1-Methylpropylphenyl, 2-Methylpropylphenyl, 1,1-Dimethylethylphenyl, Methylnaphthyl, Ethylnaphthyl, Propylnaphthyl, 1-Methylethylnaphthyl, Butylnaphthyl, 1-Methylpropylnaphthyl, 2-Methylpropylnaphthyl oder 1,1-Dimethylethylnaphthyl genannt. Als substituierte $C_7$-$C_{16}$-Alkylarylreste seien beispielhaft die entsprechenden, mit den nachstehend beschriebenen Substituenten, substituierten $C_7$-$C_{16}$-Alkylarylreste genannt.

Als unsubstituierte, verzweigtkettige oder unverzweigtkettige $C_5$-$C_{18}$-Alkylheteroarylreste seien für $R^3$, $R^4$ oder $R^5$ beispielsweise mit $C_1$-$C_6$-Alkylresten, wie vorstehend für $R^2$ beschrieben, substituierte $C_4$-$C_{12}$-Heteroarylreste, wie vorstehend für X beschrieben, genannt. Als substituierte $C_5$-$C_{18}$-Alkylheteroarylreste seien beispielhaft die entsprechenden, mit den nachstehend beschriebenen Substituenten, substituierten $C_5$-$C_{18}$-Alkylheteroarylreste genannt.

Als unsubstituierte oder substituierte $C_6$-$C_{14}$-Arylreste oder $C_4$-$C_{12}$-Heteroarylreste für $R^3$, $R^4$ oder $R^5$ seien beispielsweise die vorstehend für X beschriebenen $C_6$-$C_{14}$-Arylreste bzw. $C_4$-$C_{12}$-Heteroarylreste genannt, die gegebenenfalls mit einem oder mehreren der nachstehend beschriebenen Substituenten oder weiteren gesättigten oder ungesättigten nicht aromatischen Ringen oder Ringsystemen substituiert sein können. Bevorzugt sind Phenyl, Methoxyphenyl, p-Chlorphenyl oder Furyl oder die entsprechenden substituierten Verbindungen.

Unter unsubstituierten olefinischen oder aromatischen Acylresten werden für $R^3$, $R^4$ oder $R^5$ $C_1$-$C_{15}$-Acylreste, wie $C_1$-$C_4$-Alkylacylreste, wie Formyl, Acetyl, Propionyl oder Butyryl oder $C_7$-$C_{15}$-Arylacylreste wie Benzoyl oder Naphthoyl verstanden. Als substituierte Acylreste seien beispielhaft die entsprechenden, mit den nachstehend beschriebenen Substituenten, substituierten Acylreste genannt.

Als Substituenten der genannten Reste von $R^3$, $R^4$ und $R^5$ kommen prinzipiell alle denkbaren Substituenten in Frage, beispielsweise ein oder mehrere Substituenten wie Halogen, wie Fluor, Chlor, Brom oder Iod oder Substituenten wie Cyano, Nitro, Amino, Hydroxy, Alkyl, Aryl, Cycloalkyl, Aryl, Heteroaryl, Alkoxy, Benzyloxy, Phenyl oder Benzyl.

$R^6$ bedeutet Wasserstoff oder einen unsubstituierten oder substituierten, verzweigten oder unverzweigten $C_1$-$C_6$-Alkyl-, $C_1$-$C_4$-Alkoxy-, $C_3$-$C_6$-Cycloalkyl-, $C_6$-$C_{14}$-Aryl- oder $C_4$-$C_{12}$-Heteroarylrest.

Als unsubstituierte $C_1$-$C_6$-Alkylreste seien für $R^6$ beispielsweise die vorstehend für $R^2$ beschriebenen, unsubstituierten $C_1$-$C_6$-Alkylreste, vorzugsweise Methyl oder Ethyl, genannt. Als substituierte $C_1$-$C_6$-Alkylreste seien beispielhaft die entsprechenden, mit den nachstehend beschriebenen Substituenten, substituierten $C_1$-$C_6$-Alkylreste, vorzugsweise Chlormethyl, 2-Chlorethyl oder Methoxymethyl, genannt.

Als unsubstituierte $C_1$-$C_4$-Alkoxyreste seien für $R^6$ beispielsweise die vorstehend für $R^2$ beschriebenen unsubstituierten $C_1$-$C_4$-Alkoxyreste, vorzugsweise Methoxy oder Ethoxy, genannt. Als substituierte $C_1$-$C_4$-Alkoxyreste seien beispielhaft die entsprechenden, mit den nachstehend beschriebenen Substituenten, substituierten $C_1$-$C_4$-Alkoxyreste, wie Chlormethoxy genannt.

Als unsubstituierte $C_3$-$C_6$-Cycloalkylreste seien für $R^6$ beispielsweise die vorstehend für $R^2$ beschriebenen unsubstituierten $C_3$-$C_6$-Cycloalkylreste, vorzugsweise Cyclopentyl oder Cyclohexyl, genannt. Die Cycloalkylreste können auch Heteroatome wie S, N und O im Ring enthalten. Als substituierte $C_3$-$C_6$-Cycloalkylreste seien beispielhaft die entsprechenden, mit den nachstehend beschriebenen Substituenten, substituierten $C_3$-$C_6$-Cycloalkylreste genannt.

Als unsubstituierte $C_6$-$C_{14}$-Arylreste oder $C_4$-$C_{12}$-Heteroarylreste seien für $R^6$ beispielsweise die vorstehend für X beschriebenen $C_6$-$C_{14}$-Arylreste bzw. $C_4$-$C_{12}$-Heteroarylreste, vorzugsweise Phenyl, Naphthyl oder Furyl, genannt. Als substituierte $C_6$-$C_{14}$-Arylreste bzw. $C_4$-$C_{12}$-Heteroarylreste seien beispielhaft die entsprechenden, mit den nachstehend beschriebenen Substituenten, substituierten $C_6$-$C_{14}$-Arylreste bzw. $C_4$-$C_{12}$-Heteroarylreste, vorzugsweise p-Chlorphenyl oder p-Methoxyphenyl, genannt.

Als Substituenten der genannten Reste von $R^6$ kommen beispielsweise ein oder mehrere Substituenten wie Halogen, wie Fluor, Chlor, Brom oder Iod, vorzugsweise Chlor oder Substituenten wie Cyano, Nitro, Amino oder Hydroxy oder Substituenten wie $C_1$-$C_6$-Alkyl, oder $C_1$-$C_4$-Alkoxy, wie jeweils vorstehend für $R^2$ beschrieben, in Frage.

$R^7$ bedeutet Wasserstoff oder einen unsubstituierten oder substituierten, verzweigten oder unverzweigten $C_1$-$C_6$-Alkyl-, $C_1$-$C_4$-Alkoxy- oder $C_3$-$C_6$-Cycloalkylrest.

Als unsubstituierte $C_1$-$C_6$-Alkylreste seien für $R^7$ beispielsweise die vorstehend für $R^2$ beschriebenen, unsubstituierten $C_1$-$C_6$-Alkylreste, vorzugsweise Methyl oder Ethyl, genannt. Als substituierte $C_1$-$C_6$-Alkylreste seien beispielhaft die entsprechenden, mit den nachstehend beschriebenen Substituenten, substituierten $C_1$-$C_6$-Alkylreste, vorzugsweise Chlormethyl, 2-Chlorethyl oder Methoxymethyl, genannt.

Als unsubstituierte $C_1$-$C_4$-Alkoxyreste seien für $R^7$ beispielsweise die vorstehend für $R^2$ beschriebenen unsubstituierten $C_1$-$C_4$-Alkoxyreste, vorzugsweise Methoxy oder Ethoxy, genannt. Als substituierte $C_1$-$C_4$-Alkoxyreste seien beispielhaft die entsprechenden, mit den nachstehend beschriebenen Substituenten, substituierten $C_1$-$C_4$-Alkoxyreste, wie Chlormethoxy, genannt.

Als unsubstituierte $C_3$-$C_6$-Cycloalkylreste seien für $R^7$ beispielsweise die vorstehend für $R^2$ beschriebenen

unsubstituierten $C_3$-$C_6$-Cycloalkylreste, vorzugsweise Cyclopentyl oder Cyclohexyl, genannt. Die Cycloalkylreste können auch Heteroatome wie S, N und O im Ring enthalten. Als substituierte $C_3$-$C_6$-Cycloalkylreste seien beispielhaft die entsprechenden, mit den nachstehend beschriebenen Substituenten, substituierten $C_3$-$C_6$-Cycloalkylreste genannt.

Als Substituenten der genannten Reste von $R^7$ kommen beispielsweise ein oder mehrere Substituenten wie Halogen, wie Fluor, Chlor, Brom oder Iod, vorzugsweise Chlor, oder Substituenten wie Cyano, Nitro, Amino oder Hydroxy, oder Substituenten wie $C_1$-$C_6$-Alkyl oder $C_1$-$C_4$-Alkoxy, wie jeweils vorstehend für $R^2$ beschrieben, in Frage.

In dieser besonders bevorzugten Ausführungsform des erfindungsgemäßen Verfahrens werden die stereoisomeren Carbonsäureester der allgemeinen Formel I hergestellt, indem man einen racemischen Carbonsäureester der allgemeinen Formel II

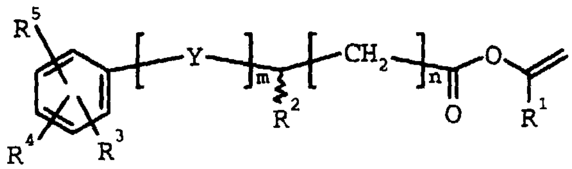

(II),

wobei

$R^1$ Wasserstoff oder $C_1$-$C_4$-Alkyl bedeutet,

mit einem racemischen Alkohol der allgemeinen Formel III

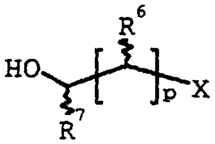

(III)

in Gegenwart einer Carboxylesterhydrolase (EC 3.1.1) umsetzt. $R^1$ bedeutet Wasserstoff oder $C_1$-$C_4$-Alkyl, wie Methyl, Ethyl, Propyl, iso-Propyl, n-Butyl, iso-Butyl, tert. -Butyl, vorzugsweise Wasserstoff, oder Methyl.

Bevorzugte racemische Carbonsäureester der allgemeinen Formel II, die sich für das erfindungsgemäße Verfahren besonders gut eignen, sind racemische Carbonsäureester der allgemeinen Formel IIa

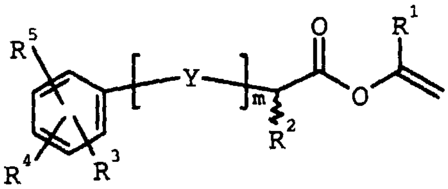

(IIa)

wobei die Variable m und die Reste Y, $R^2$, $R^3$, $R^4$ und $R^5$ die vorstehend beschriebene Bedeutung haben und $R^1$ Wasserstoff oder Methyl bedeutet, wie beispielsweise

(RS)-2-Phenylpropionsäurevinylester,
(RS)-2-Phenylpropionsäure-1-methylvinylester,
(RS)-2-Phenylbutansäurevinylester,
(RS)-2-Phenylbutansäure-1-methylvinylester,
(RS)-2-(4-Chlorphenyl)propionsäurevinylester,
(RS)-2-(4-Chlorphenyl)propionsäure-1-methylvinylester,
(RS)-2-(4-Chlorphenyl)butansäurevinylester,
(RS)-2-(4-Chlorphenyl)butansäure-1-methylvinylester,
(RS)-2-(4-Methylphenyl)propionsäurevinylester,
(RS)-2-(4-Methylphenyl)propionsäure-1-methylvinylester,

(RS)-2-(4-Methylphenyl)butansäurevinylester,
(RS)-2-(4-Methylphenyl)butansäure-1-methylvinylester
(RS)-2-(4-*iso*Butylphenyl)propionsäurevinylester,
(RS)-2-(4-*iso*Butylphenyl)propionsäure-1-methylvinylester,
(RS)-2-(4-*iso*Butylphenyl)butansäurevinylester,
(RS)-2-(4-*iso*Butylphenyl)butansäure-1-methylvinylester,
(RS)-2-(4-Benzoylphenyl)propionsäurevinylester,
(RS)-2-(4-Benzoylphenyl)propionsäure-1-methylvinylester,
(RS)-2-(4-Benzoylphenyl)butansäurevinylester,
(RS)-2-(4-Benzoylphenyl)butansäure-1-methylvinylester,
(RS)-2-(6-Methoxy-2-naphthyl)propionsäurevinylester,
(RS)-2-(6-Methoxy-2-naphthyl)propionsäure-1-methylvinylester,
(RS)-2-(6-Methoxy-2-naphthyl)butansäurevinylester,
(RS)-2-(6-Methoxy-2-naphthyl)butansäure-1-methylvinylester,
(RS)-2-Benzylpropionsäurevinylester,
(RS)-2-Benzylpropionsäure-1-methylvinylester,
(RS)-2-Benzylbutansäurevinylester,
(RS)-2-Benzylbutansäure-1-methylvinylester,
(RS)-2-(4-Chlorbenzyl)propionsäurevinylester,
(RS)-2-(4-Chlorbenzyl)propionsäure-1-methylvinylester,
(RS)-2-(4-Chlorbenzyl)butansäurevinylester,
(RS)-2-(4-Chlorbenzyl)butansäure-1-methylvinylester,
(RS)-2-Phenoxypropionsäurevinylester,
(RS)-2-Phenoxypropionsäure-1-methylvinylester,
(RS)-2-(4-Chlorphenoxy)-propionsäurevinylester,
(RS)-2-(4-Chlorphenoxy)propionsäure-1-methylvinylester,
(RS)-2-(2,4-Dichlorphenoxy)propionsäurevinylester,
(RS)-2-(2,4-Dichlorphenoxy)propionsäure-1-methylvinylester,
(RS)-2-(3-Chlorphenoxy)propionsäurevinylester oder
(RS)-2-(3-Chlorphenoxy)propionsäure-1-methylvinylester.

Bei bevorzugten racemischen Alkoholen der allgemeinen Formel III, die sich für das erfindungsgemäße Verfahren besonders gut eignen, bedeutet der Rest X einen substituierten oder unsubstituierten $C_2$-$C_{10}$-Alkenyl-, $C_2$-$C_{10}$-Alkinyl-, $C_4$-$C_{12}$-Hetaryl- oder $C_6$-$C_{14}$-Arylrest, während die Variable p und die Reste $R^6$ und $R^7$ die vorstehend beschriebene Bedeutung haben.

Besonders bevorzugte racemische Alkohole der allgemeinen Formel III sind racemische Alkohole der Formel IIIa

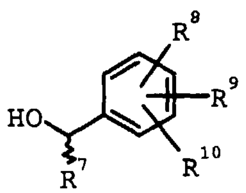

(IIIa)

wobei $R^7$ die vorstehend beschriebene Bedeutung hat und $R^8$, $R^9$ und $R^{10}$ unabhängig voneinander die vorstehend für $R^3$, $R^4$ und $R^5$ beschriebene Bedeutung haben.

Beispielhaft seien (RS)1-Phenylethanol, (RS)1-Phenylpropanol, (RS)1-(4-Chlorphenyl)ethanol, (RS)1-(4-Chlorphenyl)propanol, (RS)2-Chlor-1-phenylethanol, (RS)3-Chlor-1-Phenylpropanol, (RS)2-Chlor-1-(4-Chlorphenyl)ethanol, (RS)3-Chlor-1-(4-Chlorphenyl)propanol, (RS)2-Chlor-1-(3-Chlorphenyl)ethanol, (RS)3-Chlor-1-(3-Chlorphenyl)propanol, (RS)2-Chlor-1-(2-Chlorphenyl)ethanol, (RS)3-Chlor-1-(2-Chlorphenyl)propanol, (RS)1-(4-Nitrophenyl)ethanol, (RS)1-(4-Nitrophenyl)propanol, (RS) 1-Naphthylethanol, (RS)1-Naphthylpropanol, (RS)1-(6-Methoxynaphthyl)ethanol, (RS)1-(6-Methoxynaphthyl)propanol, (RS)2-Chlor-1-naphthylethanol, (RS)3-Chlor-1-naphthylpropanol, (RS)2-Chlor-1-(6-methoxynaphthyl)ethanol, (RS)3-Chlor-1-(6-methoxynaphthyl)propanol, (RS)1-(4-Methylphenyl)ethanol, (RS)1-(4-Methylphenyl)propanol, (RS)2-Chlor-1-(4-Methylphenyl)ethanol, (RS)3-Chlor-1-(4-Methylphenyl)propanol, (RS)1-(4-Ethylphenyl)ethanol, (RS)1-(4-Ethylphenyl)propanol, (RS)2-Chlor-1-(4-ethylphenyl)ethanol, (RS)3-Chlor-1-(4-ethylphenyl)propanol, (RS)1-(4-Methoxyphenyl)ethanol, (RS)1-(4-Methoxyphenyl)propanol, (RS)2-Chlor-1-(4-methoxyphenyl)ethanol, (RS)3-Chlor-1-(4-methoxyphenyl)propanol, (RS)1-(2-Methylphenyl)ethanol, (RS)1-(2-Methylphenyl)propanol, (RS)2-Chlor-1-(2-Methylphenyl)ethanol, (RS)3-Chlor-1-(2-Methylphenyl)propanol, (RS)1-(2-Ethylphenyl)ethanol, (RS)1-(2-Ethylphenyl)propanol, 2-Chlor-1-(2-ethylphenyl)ethanol, (RS)3-Chlor-1-(2-ethylphenyl)propanol, (RS)1-(2-Methoxyphenyl)ethanol, (RS)1-(2-Methoxyphenyl)propanol, 2-Chlor-1-(2-methoxyphenyl)ethanol, (RS)3-Chlor-1-(2-methoxyphenyl)propanol, (RS)1-(3-Methylphenyl)ethanol, (RS)1-(3-Methylphenyl)propanol, (RS)2-Chlor-1-(3-Methylphenyl)ethanol, (RS)3-Chlor-1-(3-Methylphenyl)propanol, (RS)1-(3-Ethylphenyl)ethanol, (RS)1-(3-Ethylphenyl)propanol, (RS)2-Chlor-1-(3-ethylphenyl)ethanol, (RS)3-Chlor-1-(3-ethylphenyl)propanol, (RS)1-(3-Methoxyphenyl)ethanol, (RS)1-(3-Methoxyphenyl)propanol, (RS)2-Chlor-1-(3-methoxyphenyl)ethanol, (RS)3-Chlor-1-(3-methoxyphenyl)propanol oder (RS)1-(1,3)-Benzodioxolethanol genannt.

Im erfindungsgemäßen Verfahren werden der racemische Alkohol und der racemische Carbonsäureester vorteilhaft in äquimolaren Mengen eingesetzt.

Für das erfindungsgemäße Verfahren sind prinzipiell alle Carboxylesterhydrolasen (EC. 3.1.1), wie mikrobielle, tierische oder pflanzliche Carboxylesterhydrolasen, geeignet. Die Carboxylesterhydrolasen können als freie Enzyme oder als Enzymformulierungen, beispielsweise in immobilisierter Form, verwendet werden. Die Reaktion kann auch in Gegenwart der gesamten Organismen oder Rohextrakten der Organismen durchgeführt werden. Vorteilhaft werden im erfindungsgemäßen Verfahren Carboxylesterasen (Esterasen (EC. 3.1.1.1)) oder Lipasen (EC. 3.1.1.3) als Carboxylesterhydrolasen verwendet. Vorzugsweise werden bakterielle, pilzliche, tierische oder pflanzliche Lipasen oder Carboxylesterasen verwendet. Beispielhaft seien als Carboxylesterasen Carboxylesterasen aus Bacterium subtilis, Bacterium stearothermophilus, Bacterium thermoglucosidasius, Candida lipolytica, Mucor Miehei, Pferdeleber, Schweineleber, Saccharomyces cerevisiae, Thermoanaerobium brockii oder Elektrophorus electricus genannt.

Besonders bevorzugt werden Lipasen verwendet. Als Lipasen sind Schweinepankreaslipase (PPL) oder Weizenkeimlipase sowie bakterielle oder pilzliche Lipasen geeignet, die aus den Gattungen Aspergillus, Arthrobacter, Alcaligenes, Bacillus, Brevibacterium, Pseudomonas, Burkholderia, Chromobacterium, Candida, Fusarium, Geotrichum, Humicola, Mucor, Pichia, Penicillium, Rhizomucor, Rhizopus oder Thermus isolierbar sind. Besonders vorteilhaft geeignet sind Lipasen aus den Gattungen und Arten Arthrobacter sp., Alcaligenes sp., Bacillus cereus, Bacillus subtilis, Bacillus coagulans, Burkholderia plantarii, Brevibacterium ammoniagenes, Pseudomonas aeruginosa, Pseudomonas cepacia, Pseudomonas fluorescens, Pseudomonas putida, Pseudomonas sp., Chromobacterium viscosum, Aspergillus niger, Aspergillus oryzae, Candida antarctica, Candida cylindracea, Candida rugosa, Candida lipolytica, Candida utilis, Fusarium solani, Geotrichum candidum, Humicola lanuginosa, Mucor javanicus, Mucor japonicus, Mucor miehei, Mucor sp., Penicillium acylase, Penicillium roquefortii, Pichia miso, Rhizopus nigricans, Phizopus oryzae, Rhizopus arrhizus, Rhizopus delemar, Rhizopus niveus, Rhizopus sp., Rhizomucor miehei, Thermus aquaticus, Thermus flavus oder Thermus thermophilus.

Auch kommerziell erhältliche Lipasen oder Formulierungen dieser Lipasen, die beispielsweise von den Firmen Amano, Novo oder Boehringer Mannheim erhältlich sind, sind für das erfindungsgemäße Verfahren geeignet. Bevorzugt sind die Lipasen aus Candida antartica, die in zwei Isoformen A oder B oder deren Gemisch erhältlich ist, oder Lipasen aus Candida cylindracea. Diese Enzyme eignen sich als freie Enzyme oder als Enzymformulierungen beispielsweise als Lipase Chirazyme L2 von der Firma Boehringer Mannheim oder als Novozym 435 der Firma Novo für das erfindungsgemäße Verfahren. Besonders bevorzugt ist die Candida antartica Lipase B (= CAL-B).

Beim erfindungsgemäßen Verfahren entstehen stereoisomere Carbonsäureester, wobei mindestens ein Stereoisomer in einem Überschuß vorliegt. Vorteilhafterweise entsteht ein Stereoisomer in einem großen Überschuß, so daß die Reaktionsmischung nach erfolgter Umsetzung als Hauptprodukte das Überschuß-Stereoisomer, das nicht oder nur gering umgesetztes Carbonsäureester-Enantiomer und das nicht oder nur gering umgesetztes Alkohol-Enantiomer enthält.

Das erfindungsgemäße Verfahren wird vorteilhaft in Gegenwart von mindestens einem organischen Lösungsmittel durchgeführt, kann aber auch ohne Anwesenheit eines Lösungsmittels durchgeführt werden. In diesem Fall dient der racemische Alkohol der allgemeinen Formel III als Lösungsmittel. Als Lösungsmittel kommen alle organischen Lösungsmittel in Frage, die die Edukt- und Produktlöslichkeit steigern können und die Reaktionszeit und die Enantioselektivität positiv beeinflussen. Bei Verwendung eines organischen Lösungsmittels kann das Lösungsmittel einen Effekt auf die Enantioselektivität ausüben. Je nach Lösungsmittel kann die Enantioselektivität erhöht oder erniedrigt oder auch umgedreht werden. Das für die jeweiligen Edukte und Enzyme optimale Lösungsmittel kann durch einfache Vorversuche ermittelt werden. Bevorzugt werden als organische Lösungsmittel aprotische Lösungsmittel wie Toluol, Hexan oder Benzol oder polar aprotische Lösungsmittel wie DMSO, DMF oder N-Methylpyrrolidon für das erfindungsgemäße Verfahren verwendet.

Das erfindungsgemäße Verfahren läßt sich bei Temperaturen zwischen -50°C und +100°C durchführen. Bei Verwendung thermostabiler Enzyme können auch höhere Reaktionstemperaturen erreicht werden (siehe beispielsweise Ikeda et al, Molecular cloning of extremely thermostable esterase gene from hyperthermophilic archaeon Pyrococcus furiosus in Escherichia coli, Biotechnol. Bioeng., 57, 1998: 624-629). Im Bereich von 0°C oder darunter nimmt

die Reaktionsgeschwindigkeit deutlich ab. Eine Reaktion in diesem Bereich ist jedoch prinzipiell möglich, wie Sakai et al. zu entnehmen ist (Enhancement of the enantioselectivity in lipase-catalysed kinetic resolutions of 3-phenyl-2H-azirine-2-methanol by lowering the temperature to -40 degree, J. Org. Chem., 62, 1997: 4906-4907). Bevorzugt wird das Verfahren zwischen 0°C und 90°C, besonders bevorzugt zwischen 10°C und 80°C, durchgeführt.

Die Reaktionszeiten bis zur Einstellung des Gleichgewichts betragen typischerweise 1 h bis 60 h, vorzugsweise 2 bis 40 h.

Das erfindungsgemäße Verfahren kann kontinuierlich oder diskontinuierlich durchgeführt werden. Für die kontinuierliche Verfahrensdurchführung leitet man beispielsweise in an sich bekannter Weise in einem Reaktor durch eine Schüttschicht aus freier oder immobilisierter Carboxylesterhydrolase eine flüssige mobile Phase. Die mobile Phase kann entweder eine Lösung der racemischen Edukte in einem vorstehend erwähnten organischen Lösungsmittel bzw. das flüssige Eduktgemisch ohne Lösungsmittel sein. Die Durchflußrate ist nicht kritisch und richtet sich nach verfahrenstechnischen Gesichtspunkten wie Höhe, Durchmesser und Korngröße der Schüttschicht sowie nach der Ausgestaltung des Reaktors. Als Reaktoren für das kontinuierliche Verfahren verwendet man vorzugsweise die für kontinuierliche, heterogenkatalytische Prozesse (Fluid/Feststoff-Reaktionen) üblichen Reaktoren (J. Hagen, Chemische Reaktionstechnik, VCH, Weinheim 1992, S. 165-169). Beispielhaft seien Wirbelschichtreaktoren und Festbettreaktoren, wie Rohrreaktor, Säulenreaktor, Vollraumreaktor, Hordenreaktor, Rohrbündelreaktor und Flachbett-Kontaktofen, genannt.

In der diskontinuierlichen Verfahrensdurchführung wird die Carboxylesterhydrolase in an sich bekannter Weise in einem Reaktor in einer Lösung der racemischen Edukte in einem vorstehend erwähnten organischen Lösungsmittel bzw. in dem flüssigen Eduktgemisch mit oder ohne organischem Lösungsmittel suspendiert und die Suspension durchmischt. Als Reaktoren für das diskontinuierliche Verfahren verwendet man vorzugsweise die für diskontinuierliche, heterogenkatalytische Prozesse (Fluid/Feststoff-Reaktionen) üblichen Reaktoren mit Schüttel-, Misch- oder Rührvorrichtung. Beispielhaft sei der Rührkessel und sich davon ableitende Ausgestaltungen sowie Reaktionsgefäße mit Schüttelvorrichtung erwähnt.

Die Abtrennung und Isolierung des stereoisomeren Carbonsäureesters, der nach Durchführung des erfindungsgemäßen Verfahrens im Überschuß im Produktgemisch vorliegt, kann in an sich bekannter Weise durch physikalische oder chemische Reinigungs- und Trennverfahren durchgeführt werden. Beispielhaft seien Extraktionsverfahren, Chromatographieverfahren, wie beispielsweise Chromatographie über Kieselgel oder HPLC über chirale Säulen, Kristallisationsverfahren, wie Kristallisation und Umkristallisation aus organischen Lösungsmitteln, wie n-Hexan, Toluol oder Methylenchlorid oder Rektifikationsverfahren wie Destillation genannt.

Darüber hinaus eignet sich das erfindungsgemäße Verfahren zu einer Racemattrennung von racemischen Alkoholen und racemischen Carbonsäureestern in einer Eintopfreaktion.

Das erfindungsgemäße Verfahren weist gegenüber dem Stand der Technik folgende Vorteile auf:

- Der Umsatz wird bei deutlich kürzeren Reaktionszeiten auf ca. 50 % gesteigert.
- Dabei werden gleichzeitig hohe Enantioselektivitäten und Dia-stereomerenüberschüsse erhalten.
- Bei den erhaltenen Produkten läßt sich direkt ohne weitere Reinigung der Diastereomerenüberschuß durch Umkristallisation weiter steigern.

Die nachstehenden Beispiele erläutern die Erfindung:

Allgemeine Versuchsbedingungen

Wenn nicht anders beschrieben, wurden die $^1$H-NMR-Spektren bei 250,1 und 500,1 MHz und die $^{13}$C-NMR-Spektren bei 62,9 und 125,7 MHz in $CDCl_3$ mit Tetramethylsilan als internem Standard aufgenommen. Die kursiv angegebenen Signale entsprechen dem Unterschuß-Diastereomer. Zur Bestätigung der chemischen Identität wurden die Carbonsäureester auch chemisch hergestellt.

Die Bestimmung der absoluten Konfiguration und des Enantiomeren- und Diastereomerenüberschusses erfolgte wie nachstehend beschrieben.

Der Enantiomerenüberschuß der Carbonsäureester wurde per GC über eine Heptakis-(2,3-di-O-acetyl-6-O-TBDMS)-β-cyclodextrin-Säule (25 m x 0,25 mm, Prof. W.A. König, Universität Hamburg) bestimmt. Der Enantiomerenüberschuß des Alkohols wurde per GC über eine Heptakis-(2,3,6-tri-O-methyl)-β-cyclodextrin-Säule (50 m x 0,25 mm, CS-Chromatographie-Service, Langerwehe) bestimmt. Die Bestimmung des Diastereomerenüberschusses der Carbonsäureester und des Umsatzes erfolgte per GC über eine Optima 5 Säule (25 m x 0,25 mm; Macherey & Nagel, Düren). Der Diastereomerenüberschuß wurde zusätzlich durch Vergleich der Signalintensitäten der Diastereomere im NMR-Experiment verifiziert. Zusätzlich wurde der Enantiomerenüberschuß der Carbonsäure- und Alkoholkomponente der hergestellten stereoisomeren Carbonsäureester nach der chemischen Hydrolyse bestimmt. Die Hydrolyse erfolgte durch Lösen des Carbonsäureesters (3 mg) in Heptan (200 µl), Zugabe einer methanolischen Kaliumhydroxid-Lösung (2N, 90 µl) und anschließendem Rühren (1 min). Proben aus der organischen Phase, enthaltend den aus der Alkoholkomponente des Carbonsäureester entstandenen Alkohol und den aus der Carbonsäurekomponente entstandenen Carbonsäuremethylester, wurden per GC durch Verwendung der vorstehend beschriebenen chiralen Säulen analysiert. Die Enantiomerenüberschüsse wurden durch Messen der optischen Drehwerte an einem Perkin Elmer Polarimeter 241 verifiziert. Die absolute Konfiguration wurde durch Vergleich mit den Drehwerten von optisch reinen Proben zugeordnet.

Die Enantioselektivität wurde mit der Formeln

$$E = [\ln\{(1-c) \times (1-ee_s)\}]/[\ln\{(1-c) \times (1+ee_s)\}], \text{ bzw.}$$

$E = [\ln\{1-cx(1+ee_p)\}]/[\ln\{1-cx(1-ee_p)\}]$ jeweils für den Alkohol- und die Carbonsäurekomponente bestimmt, wobei

c den Umsatz,
$ee_s$ den Enantiomerenüberschuß eines Substratenantiomers und
$ee_p$ den Enantiomerenüberschuß eines Produktenantiomers

darstellt (Sih et al., J. Am. Chem. Soc. 1982, 104, 7294-7299).

Als immobilisierte Lipase wurde, wenn nicht anders beschrieben, Lipase aus Candida antarctica (SP435) (CAL-B; Chirazyme L-2, c.-f., C2, 5000 U/g) der Firma Boehringer Mannheim, Penzberg, verwendet. Alle Chemikalien wurden, wenn nicht anders beschrieben, von Fluka, Buchs, Schweiz, bezogen. Die Lösungsmittel und die Lipase wurden vor Ihrer Verwendung über aktiviertem Molsieb (4Å) getrocknet.

Beispiel 1

Herstellung der racemischen Carbonsäureester

1.1 Herstellung der racemischen Carbonsäurevinylester

Allgemeine Arbeitsvorschrift:

Die racemischen Carbonsäurevinylester wurden nach der von Wang et al. (J. Am. Chem. Soc. 1988, 110, 7200) beschriebenen Methode hergestellt. 500 mg der Carbonsäure und $HgOAc_2$ (70 mg, 0,22 mmol) wurden in Vinylacetat (10 ml) gelöst. Die Lösung wurde 30 min. bei Raumtemperatur (23°C) gerührt, anschließend wurden 0,1 ml $H_2SO_4$ (konz.) zugegeben, die Lösung 6 Stunden refluxiert und auf Raumtemperatur abgekühlt. Danach wurden 400 mg NaOAc zum Quenchen des Katalysators zugegeben. Die Lösung wurde gefiltert und aufkonzentriert. Die Rohprodukte wurden anschließend über Silicagelchrormatographie (Petrolether:$Et_2O$, 20:1) aufgereinigt. Alle Vinylester wurden als farblose Flüssigkeiten erhalten, die keiner weiteren Aufreinigung bedurften. Optisch reine Vinylester wurden in kleineren Mengen unter Verwendung von 60 bis 120 mg optisch reiner Carbonsäuren synthetisiert.

1.1.1 (RS)-2-Phenylbuttersäurevinylester ((RS)IIaa):

Die Herstellung erfolgte nach der oben beschriebenen Methode unter Verwendung von 500 mg (= 3,05 mmol) der entsprechenden racemischen Carbonsäure [(RS)2-Phenylbuttersäure]. Es wurden 290 mg (RS)IIaa (1,52 mmol, 50 %) erhalten.

Analytische Daten:

C, 75,80; H, 7,41.; ber. für $C_{12}H_{14}O_2$: C, 75,76; H, 7,42;
$^1$H-NMR (500,1 MHz; $CDCl_3$) δ[ppm]= 0,91 (t, J = 7,4, 3H), 1,80-2,16 (m, 2H), 3,51 (t, J = 7,7, 1H), 4,54 (dd, J = 6,32, 1,6, 1H), 4,85 (dd, J = 14,0, 1,7, 1H), 7,23-7,34 (m, 6H);
$^{13}$C-NMR (125,8 MHz; $CDCl_3$) δ[ppm] = 12,08, 26,62, 53,17, 97,90, 127,41, 128,00, 128,67, 138,28, 141,32, 171,12;
IR (KBr)/$cm^{-1}$: 3080w, 3050w, 3015w, 2955vs, 2920s, 2860w, 1750vs, 1640vs, 1595w, 1475s, 1447s, 1130br, 860s,

720s, 680vs.

1.1.2 R-(-)-2-Phenylbuttersäurevinylester ((R-(-)-IIaa):

120 µl R-(-)-2-Phenylbuttersäure (126,6 mg, 0,77 mmol) erbrachten 44 mg R-(-)-IIaa (0,23 mmol, 30 %); $[\alpha]_D^{22}$ = -23,9 (c 0,664, $CHCl_3$).

1.1.3 (RS)-2-Phenylpropionsäurevinylester ((RS)IIab):

420 µl (RS)-2-Phenylpropionsäure (460,7 mg, 3,07 mmol) erbrachten 210 mg (RS)IIab (1,19 mmol, 39 %).

Analytische Daten:

C, 74,73; H, 6,93; ber. für $C_{11}H_{12}O_2$; C, 74,98; H, 6,86;
$^1$H-NMR (500,1 MHz; $CDCl_3$) δ [ppm] = 1,53 (d, J = 7,2, 3H), 3,79 (q, J = 7,1, 1H), 4,54 (d, J = 6,18; 1H), 4,77 (d, J = 14,0, 1H), 7,23-7,35 (m, 6H);
$^{13}$C NMR (125,7 MHz; $CDCl_3$) 18,41, 45,26, 97,92, 127,36, 127,52, 128,73, 139,71, 141,36, 171,59;
IR (KBr)/cm$^{-1}$: 3080w, 3050w, 3020s, 2970s, 2920s, 2860w, 1750vs, 1640vs, 1595w, 1485s, 1445s, 1140br, 860s, 715s, 680vs.

1.1.4 R-(-)-2-Phenylpropionsäurevinylester ((R)-(-)-IIab):

60 µl R-(-)-2-Phenylpropionsäure (65,8 mg, 0,44 mmol) erbrachten 18 mg (R)-(-)-IIab) (0,10 mmol, 23 %) $[\alpha)_D^{22}$ = -34,6 (c 0, 900, EtOH).

1.1.5 (RS)-3-Phenylbuttersäurevinylester ((RS)IIb):

500 mg (RS)-3-Phenylbuttersäure (3,05 mmol) erbrachten 300 mg (RS)IIb (1,58 mmol, 52 %):

Analytische Daten:

C, 75,63; H, 7,68; ber. für $C_{12}H_{14}O_2$: C, 75,76; H, 7,42;
$^1$H NMR (250,1 MHz; $CDCl_3$) δ 1,25 (d, J = 7,0, 3H), 2,59 (m, 2H), 3,24 (m, 1H), 4,47 (dd, J = 6,3, 1,5, 1H), 4,77 (dd, J = 14,0, 1,5, 1H), 7,13-7,27 (m, 6H);
$^{13}$C NMR (62,9 MHz; $CDCl_3$) δ 21,82, 36,27, 42,60, 97,76, 126,63, 126,78, 128,66, 141,18, 145,41, 169,48;
IR (KBr)/cm$^{-1}$: 3070w, 3050w, 3010s, 2950s, 2910w, 2860w, 1750vs, 1640vs, 1595w, 1485s, 1445s, 1140br, 860s, 745s, 680vs.

1.1.6 R-(-)-3-Phenylbuttersäurevinylester (R-(-)-IIb):

120 µl R-(-)-3-Phenylbuttersäure (128,3 mg, 0,78 mmol) erbrachten 29 mg R-(-)-IIb (0,15 mmol, 19 %) $[\alpha]_D^{22}$ = -21,2 (c 1,543, 1,4-Dioxan).

Beispiel 2

Lipase-katalysierte Umsetzung von racemischen Carbonsäureestern mit racemischen Alkoholen

Beispiel 2.1

Lipase-katalysierte Umsetzung von racemischen Carbonsäurevinylestern mit racemischen Alkoholen

Allgemeine Arbeitsvorschrift:

Der racemische Carbonsäurevinylester (0,65 mmol) und der racemische Alkohol (0,65 mmol) wurden in 6

ml Toluol gelöst und bei 40°C gerührt. Die Reaktion wurde durch Zugabe von 300 mg Lipase CAL-B gestartet. Proben wurden der Reaktion entnommen, mit Toluol verdünnt und per Gaschromatographie (GC) unter Verwendung der Optima 5-Säule analysiert. Bei 50 % Umsatz wurde die Reaktion durch Abzentrifugieren der Lipase beendet. Das Produkt und nichtumgesetztes Substrat wurden durch Flash-Chromatographie über Kieselgel gereinigt.

2.1.1 Herstellung von (R)-2-Phenylbuttersäure-(R)-1-phenethylester ((R,R)Ia) durch Lipase-katalysierte Umsetzung von (RS)-2-Phenylbuttersäurevinylester ((RS)IIaa) mit (RS)-1-Phenylethanol ((RS)IIIaa)

Schema 1 zeigt die Reaktion. Als Hauptprodukte liegen nach der Umsetzung (R)-2-Phenylbuttersäure-(R)-1-phenethylester ((R,R)Ia), das aus der Abgangsgruppe Vinylethanol tautomerisiertes Ethanal, sowie die nicht umgesetzten Substrate (S)-(-)-2-Phenylbuttersäurevinylester ((S)-(+)-IIaa) und (S)-1-Phenylethanol ((S)-IIIaa) vor.

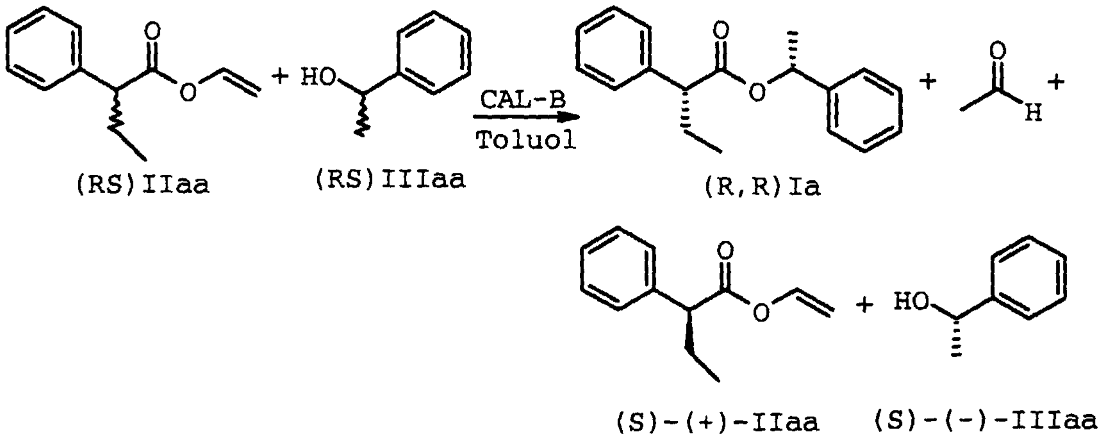

(RS)IIaa (RS)IIIaa (R,R)Ia

(S)-(+)-IIaa (S)-(-)-IIIaa

Schema 1

Die Durchführung der Umsetzung nach der vorstehend beschriebenen allgemeinen Arbeitsvorschrift lieferte nach 35 Stunden Reaktionszeit 70 mg (R,R)Ia (0,26 mmol, 40 % Ausbeute, >98 %ee (Alkoholkomponente (R)-(+)-IIIaa nach Hydrolyse), 56 %de (GC)) als farblose Flüssigkeit.

Nicht umgesetzter Vinylester (S)-(+)-IIaa: 50 mg, 0,26 mmol, 41 % Ausbeute, 58 %ee, $[\alpha]^{22}_D$ = +14,9° (c = 1,35, $CHCl_3$);
Nicht umgesetzter Alkohol (S)-(-)-IIIa: 31 mg, 0,25 mmol, 39 % Ausbeute, 94 %ee, $[\alpha]^{22}_{D}$, = -36,8° (c = 1,1, MeOH).

Die Ergebnisse und die daraus berechneten E-Werte sind in Tabelle 1 zusammengestellt.

Analytische Daten von (R,R)Ia

Elementaranalyse: C 80,42, H 7,53, ber. für $C_{18}H_{20}O_2$: C 80,56, H 7,51, O 11,92.
$^1$H-NMR (500.1 MHz; $CDCl_3$): δ 1,06 (3H, t, $J_{3,4}$ 7,4, 4-H), 1,10 (3H, t, $J_{3,4}$ 7,3, 4-H), 1,63 (3H, d, $J_{1',2'}$ 6,7, 2'-H), 1,71 (3H, d, $J_{1',2'}$ 6,5, 2'-H), 1,98-2,34 (2H, m, 3-H); 3,69 (1H, m, 2-H), 6,06 (1H, q, $J_{1',2'}$ 6,6), 7,31-7,53 (10 H, m, Ph-H);
$^{13}$C-NMR (125,8 MHz; $CDCl_3$): δ 12,54 (4-C), 22,36, 22,75 (2'-C), 26,90, 27,07 *(3-C)*, 54,02, 54,09 (2-C), 72,78, 72,90 (1'-C), 126,10, 126,42, 127,47, 127,50, 127,95, 128,17, 128,38, 128,46, 128,69, 128,83, 128,86, 128,89, 139,38, 139,51, 142,04, 142,10 (Ph-C), 173,72, 173,72 (1-C);
IR (KBr) [$cm^{-1}$]: 2950m, 2910m, 1740s, 1485m, 1445m, 1190s, 1150s, 1050s 1015m, 740m, 680m;

2.1.2 Herstellung von (R)-2-Phenylpropionsäure-(R)-1-phenethylester ((R,R)Ib) durch Lipasen-katalysierte Umsetzung von (RS)-2-Phenylpropionsäurevinylester ((RS)IIab) mit (RS)-1-Phenylethanol ((RS)IIIaa)

Schema 2 zeigt die Reaktion. Als Hauptprodukte liegen nach der Umsetzung (R)-2-Phenylpropionsäure-(R)-1-phenethylester ((R,R)Ib), das aus der Abgangsgruppe Vinylethanol tautomerisierte Ethanal, sowie die nicht umgesetzten Substrate (S)-(+)-2-Phenylpropionsäurevinylester ((S)-(+)-IIaa) und (S)-(-)-1-Phenylethanol ((S)-(-)-IIIaa) vor.

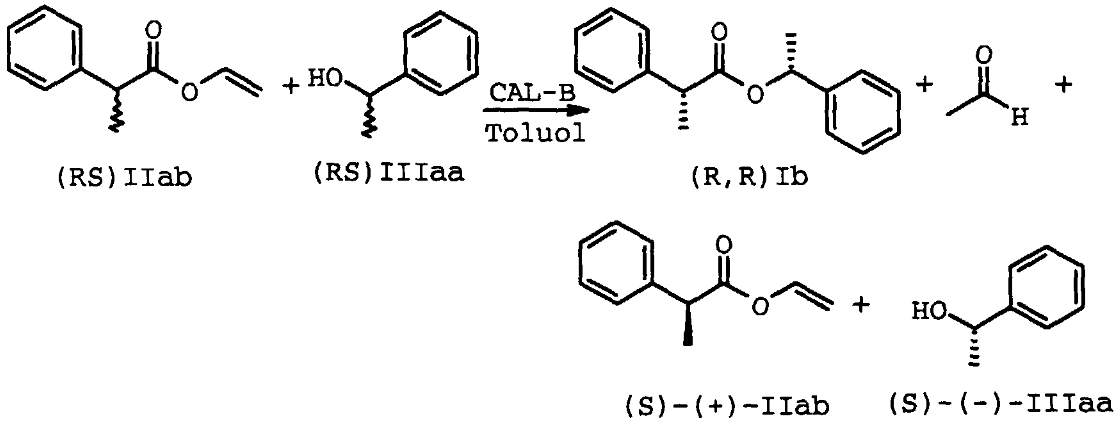


(RS)IIab (RS)IIIaa (R,R)Ib

(S)-(+)-IIab (S)-(-)-IIIaa

Die Durchführung der Umsetzung nach der vorstehend beschriebenen allgemeinen Arbeitsvorschrift lieferte nach 2,5 h Stunden Reaktionszeit 74 mg (R,R)Ib (0,29 mmol, 45 % Ausbeute, >98 %ee (Alkoholkomponente (R)-(+)-IIIaa nach Hydrolyse), 64 %de (GC), mp. 92 bis 93°C). Umkristallisation aus n-Hexan lieferte (R,R)Ib) in 98 %de (GC; >95 %de, $^1$H-NMR).

Nicht umgesetzter Vinylester (S)-(+)-IIab: 42 mg, 0,24 mmol, 36 % Ausbeute, 67 %ee, $[\alpha]^{22}_D$ = +24,9° (c = 1,19, EtOH);
Nicht umgesetzter Alkohol (S)-(-)-IIIaa: 33 mg, 0,27 mmol, 41 % Ausbeute, >98 %ee, $[\alpha]^{22}_D$ = -40,8°(c = 1.3, MeOH).

Die Ergebnisse und die daraus berechneten E-Werte sind in Tabelle 1 zusammengestellt.

Analytische Daten von (R,R)Ib:

Elementaranalyse: C 80,21, H 7,21; ber. für $C_{17}H_{18}O_2$: C 80,28, H 7,13, O 12,58.
$^1$H-NMR (250,1 MHz; $CDCl_3$): δ 1,48 (3H, d, $J_{1',2'}$ 6,6, 2'-H), 1,49 (3H, d, $J_{2,3}$ 7,2, 3-H), 3,75 (1H, q, $J_{2,3}$ = 7,2, 2-H), 5,85 (1H, q, $J_{1',2'}$ = 6,6, 1'-H), 7,06-7,31 (10H, m, Ph-H);
$^{13}$C-NMR (62,9 MHz; $CDCl_3$): δ 18,47 (3-C), 22,48 (2'-C), 45,86 (2-C), 72,59 (1'-C), 125,80, 127,16, 127,73, 128,42, 128,66, 140,57, 141,81 (Ph-H), 173,67 (1-C);
IR (KBr) [$cm^{-1}$]: 2960m, 1725s, 1325m, 1190s, 1160s, 1045m, 740m, 680s;

Tabelle 1

| Beispiel | Reaktionszeit in [h] | Umsatz in [%] | Enantiomerenüberschuß in [%ee] | | | Enantioselektivität E | | Diastereomerenüberschuß in [%de][f] |
|---|---|---|---|---|---|---|---|---|
| | | | S-II[a] | S-III[b] | R-III[c] | Säure[d] | Alkohol[e] | |
| 2.1.1 | 35 | 50 | 58 | 94 | 98 | 7 | >100 | 56 |
| 2.1.2 | 2,5 | 50 | 67 | 98 | 98 | 10 | >100 | 64 (98)[g] |

Enantiomerenüberschuß

(a) des verbliebenen (S)-Carbonsäurevinylesters der Formel II

(b) des verbliebenen (S)-Alkohols der Formel III

(c) der (R)-Alkoholkomponente der entstandenen Carbonsäureester-Stereoisomeren der Formel I nach Hydrolyse

Enantioselektivität E gegenüber

(d) der Carbonsäurekomponente

(e) der Alkoholkomponente

(f) Diastereomerenüberschuß der Summe der (R,R)- und (S,S)-Stereoisomere gegenüber der Summe der (R,S) und (S,R)-Stereoisomere

(g) nach Umkristallisation aus n-Hexan

**Patentansprüche**

1. Verfahren zur Herstellung von stereoisomeren Carbonsäureestern bestehend aus einer Säure- und einer Alkohol-Komponente mit jeweils mindestens einem chiralen Zentrum, wobei mindestens ein Stereoisomer des Carbonsäureesters in einem Überschuß vorliegt, dadurch gekennzeichnet, daß man einen racemischen Carbonsäureester mit einem racemischen Alkohol in Gegenwart einer Carboxylesterhydrolase (EC 3.1.1) umsetzt.

2. Verfahren nach Anspruch 1, dadurch gekennzeichnet, daß man als racemischen Carbonsäureester einen aktivierten racemischen Carbonsäureester verwendet.

3. Verfahren nach Anspruch 1 dadurch gekennzeichnet, daß man als racemischen Carbonsäureester einen racemischen Enolester verwendet.

4. Verfahren nach Anspruch 1 zur Herstellung von stereoisomeren Carbonsäureestern der allgemeinen Formel I

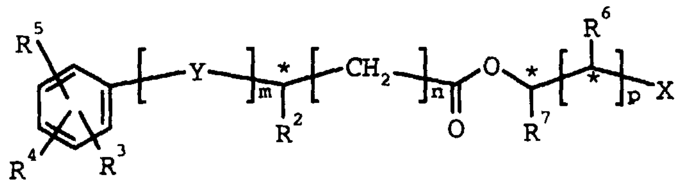 (I),

wobei die Substituenten und Variablen

| | |
|---|---|
| * | ein mögliches chirales Zentrum, |
| n, m, p | unabhängig voneinander 0 oder 1, |
| X | einen unsubstituierten oder mit F, Cl, Br, I, $NO_2$ oder $C_1$-$C_4$-Alkoxy substituierten $C_1$-$C_{10}$-Alkyl-, $C_2$-$C_{10}$-Alkenyl-, $C_2$-$C_{10}$-Alkinyl- oder $C_3$-$C_8$-Cycloalkenyl-, $C_6$-$C_{14}$-Aryl-, $C_7$-$C_{16}$-Alkylaryl-, $C_8$-$C_{16}$-Alkenylaryl-, $C_8$-$C_{16}$-Alkinylaryl-, $C_7$-$C_{18}$-Arylalkyl-, $C_8$-$C_{18}$-Arylalkenyl-, $C_8$-$C_{18}$-Arylalkinyl- oder $C_4$-$C_{12}$-Heteroarylrest oder einen substituierten $C_6$-$C_{14}$-Aryl-, $C_7$-$C_{16}$-Alkylaryl-, $C_8$-$C_{16}$-Alkenylaryl-, $C_8$-$C_{16}$-Alkinylaryl-, $C_7$-$C_{18}$-Arylalkyl-, $C_8$-$C_{18}$-Arylalkenyl-, $C_8$-$C_{18}$-Arylalkinyl oder $C_4$-$C_{12}$-Heteroarylrest, wobei jeweils zwei benachbarte Arylsubstituenten zusammen einen weiteren substituierten oder unsubstituierten aromatischen, gesättigten oder teilweise gesättigten Ring mit 5 bis 6 Atomen im Ring bilden können, der ein oder mehrere Heteroatome wie O, N, oder S enthalten kann, |
| Y | -$CH_2$-, -CO-, Sauerstoff oder Schwefel, |
| $R^2$ | einen substituierten oder unsubstituierten, verzweigten oder unverzweigten $C_1$-$C_6$-Alkyl-, $C_1$-$C_4$-Alkoxy-, $C_3$-$C_6$-Cycloalkyl-, $C_6$-$C_{14}$-Aryl- oder $C_4$-$C_{12}$-Heteroarylrest, |
| $R^3$, $R^4$, $R^5$ | unabhängig voneinander Wasserstoff, einen Hydroxy-, Halogen-, Cyano-, Nitro- oder Aminorest oder einen substituierten oder unsubstituierten, verzweigten oder unverzweigten $C_1$-$C_{10}$-Alkyl-, $C_2$-$C_{10}$-Alkenyl-, $C_2$-$C_{10}$-Alkinyl-, $C_1$-$C_{10}$-Alkoxy-, $C_2$-$C_{10}$-Alkenyloxy-, $C_2$-$C_{10}$-Alkinyloxy-, $C_3$-$C_{10}$-Cycloalkyl-, $C_3$-$C_{10}$-Cycloalkyloxy-, $C_6$-$C_{14}$-Aryl-, $C_7$-$C_{18}$-Alkylaryl-, $C_4$-$C_{12}$-Heteroaryl- oder $C_5$-$C_{16}$-Alkylheteroarylrest oder einen substituierten oder unsubstituierten olefinischen oder aromatischen Acylrest oder zwei benachbarte Substituenten $R^3$, $R^4$ oder $R^5$ zusammen einen weiteren substituierten oder unsubstituierten aromatischen, gesättigten oder teilweise gesättigten Ring mit 5 bis 6 Atomen, der ein oder mehrere Heteroatome wie O, N oder S enthalten kann, |
| $R^6$ | Wasserstoff oder einen substituierten oder unsubstituierten, verzweigten oder unverzweigten $C_1$-$C_6$-Alkyl-, $C_1$-$C_4$-Alkoxy-, $C_3$-$C_6$-Cycloalkyl-, $C_6$-$C_{14}$-Aryl - oder $C_4$-$C_{12}$-Heteroarylrest und |
| $R^7$ | Wasserstoff oder einen substituierten oder unsubstituierten, verzweigten oder unverzweigten $C_1$-$C_6$-Alkyl-, $C_1$-$C_4$-Alkoxy- oder $C_3$-$C_6$-Cycloalkylrest bedeuten, wobei |

für den Fall, daß p = O, $R^7$ nicht Wasserstoff oder X und

für den Fall, daß p = 1, entweder $R^6$ oder $R^7$ Wasserstoff, aber nicht beide gleichzeitig Wasserstoff und für den Fall, daß $R^7$ = Wasserstoff, $R^6$ nicht X und für den Fall, daß $R^7$ nicht Wasserstoff, $R^6$ Wasserstoff oder X bedeuten,

dadurch gekennzeichnet, daß man einen racemischen Carbonsäureester der allgemeinen Formel II

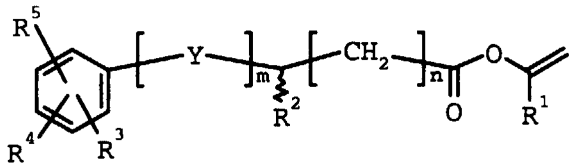

(II),

wobei

$R^1$ Wasserstoff oder $C_1$-$C_4$-Alkyl bedeutet,

mit einem racemischen Alkohol der allgemeinen Formel III

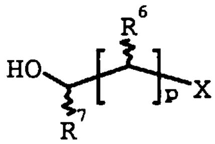

(III)

in Gegenwart einer Carboxylesterhydrolase (EC 3.1.1) umsetzt.

**5.** Verfahren nach den Ansprüchen 1 bis 4, dadurch gekennzeichnet, daß man den racemischen Alkohol und den racemischen Carbonsäureester in äquimolaren Mengen einsetzt.

**6.** Verfahren nach den Ansprüchen 1 bis 5, dadurch gekennzeichnet, daß man als Carboxylesterhydrolase eine Carboxylesterase (EC. 3.1.1.1) oder eine Lipase (EC. 3.1.1.3) verwendet.

**7.** Verfahren nach den Ansprüchen 1 bis 6, dadurch gekennzeichnet, daß man als Carboxylesterhydrolase Schweinepankreaslipase (PPL), Weizenkeimlipase oder eine bakterielle oder pilzliche Lipase verwendet, die aus den Gattungen Aspergillus, Arthrobacter, Alcaligenes, Bacillus, Brevibacterium, Pseudomonas, Burkholderia, Chromobacterium, Candida, Fusarium, Geotrichum, Humicola, Mucor, Pichia, Penicillium, Rhizomucor, Rhizopus oder Thermus isolierbar ist.

**8.** Verfahren nach den Ansprüchen 1 bis 7, dadurch gekennzeichnet, daß man als Carboxylesterhydrolase Candida antarctica Lipase (B) verwendet.

**9.** Verfahren nach den Ansprüchen 1 bis 8, dadurch gekennzeichnet, daß man die Carboxylesterhydrolase als immobilisierte Enzymformulierung verwendet.

**10.** Verfahren nach den Ansprüchen 1 bis 9, dadurch gekennzeichnet, daß man das Verfahren in Gegenwart eines organischen Lösungsmittels durchführt.